(19) Europäisches Patentamt / European Patent Office / Office européen des brevets

(11) **EP 2 523 726 B1**

(12) **EUROPÄISCHE PATENTSCHRIFT**

(45) Veröffentlichungstag und Bekanntmachung des Hinweises auf die Patenterteilung:
**16.02.2022  Patentblatt 2022/07**

(21) Anmeldenummer: **11701448.0**

(22) Anmeldetag: **11.01.2011**

(51) Internationale Patentklassifikation (IPC):
**A61N 2/02** *(2006.01)*    **A61N 2/04** *(2006.01)*

(52) Gemeinsame Patentklassifikation (CPC):
**A61N 2/006; A61N 2/02; H03K 17/136**

(86) Internationale Anmeldenummer:
**PCT/EP2011/000081**

(87) Internationale Veröffentlichungsnummer:
**WO 2011/083097 (14.07.2011 Gazette 2011/28)**

(54) **MAGNETSTIMULATION MIT FREI WÄHLBARER PULSFORM**

MAGNETIC STIMULATION HAVING A FREELY SELECTABLE PULSE SHAPE

STIMULATION MAGNÉTIQUE AU MOYEN D'UNE FORME D'IMPULSION LIBREMENT SÉLECTIONNABLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.01.2010  DE 102010004307**

(43) Veröffentlichungstag der Anmeldung:
**21.11.2012  Patentblatt 2012/47**

(73) Patentinhaber: **Technische Universität München 80333 München (DE)**

(72) Erfinder:
• **WEYH, Thomas 80637 München (DE)**
• **GÖTZ, Stefan, M. 85659 Forstern (DE)**

(74) Vertreter: **Grünecker Patent- und Rechtsanwälte PartG mbB Leopoldstraße 4 80802 München (DE)**

(56) Entgegenhaltungen:
EP-A1- 1 894 600        WO-A1-99/59674
WO-A1-2004/103173    WO-A2-2007/145838
DE-A1- 3 524 232        US-A1- 2009 018 384

• GOETZ S M ET AL: "Analysis of a novel magnetic stimulation system: Magnetic harmonic multicycle stimulation (MHMS)", BIOMEDICAL AND PHARMACEUTICAL ENGINEERING, 2009. ICBPE '09. INTERNATIONAL CONFERENCE ON, IEEE, PISCATAWAY, NJ, USA, 2. Dezember 2009 (2009-12-02), Seiten 1-6, XP031613046, ISBN: 978-1-4244-4763-3
• HIWAKI O ET AL: "Nerve Excitation Properties in Magnetic Stimulation by Trapezoidal Magnetic Fields", IEEE TRANSLATION JOURNAL ON MAGNETICS IN JAPAN, IEEE INC, NEW YORK, US, Bd. 6, Nr. 3, 1. Mai 1994 (1994-05-01), Seiten 148-152, XP011231167, ISSN: 0882-4959

EP 2 523 726 B1

**Beschreibung**

Die vorliegende Erfindung betrifft allgemein Vorrichtungen zur Reizung von Nerven- und Muskelzellen im Körpergewebe nach dem Prinzip der induktiven Magnetstimulation durch kurze starke Magnetfelderpulse die über eine Spule erzeugt werden. Weiterhin betrifft die Erfindung elektrische Leistungskreise zur Erzeugung frei wählbarer Zeitverläufe von kurzen starken Strompulsen durch die Spule um die pulsförmigen Magnetfelder zur Reizung von Nerven- oder Muskelzellen zu erzeugen.

HINTERGRUND DER ERFINDUNG

Allgemein können durch von außen einwirkende elektrische Felder im Körpergewebe bestimmte Zellen gereizt werden. Speziell Nerven- oder Muskelzellen können über die durch ein äußeres Feld angeregte Depolarisation eines Axons bzw. eines Faserbündels und der dadurch erzeugten Auslösung von Aktionspotentialen und der nachfolgenden Reizweiterleitung erregt werden. Dies geschieht dadurch, dass die elektrischen Felder im Gewebe elektrische Ströme verursachen, welche wiederum in diesen Zellen die Aktionspotentiale auslösen. Diese Art der Auslösung von Aktionspotentialen durch ein direkt am Axon einwirkendes elektrisches Feld ist ein nicht-physiologischer Prozess: In der Natur werden Aktionspotentiale im Zellkörper der Nervenzelle selbst erzeugt, nachdem über die Dendriten einlaufende Signale entsprechend verknüpft wurden.

Das von außen einwirkende elektrische Feld muss zur Auslösung eines solchen Aktionspotentials einen bestimmten zeitlichen Verlauf und eine gewisse Mindeststärke erreichen. Insbesondere reagieren unterschiedliche Zelltypen bezüglich der Auslösung eines Aktionspotentials auch unterschiedlich auf die Zeitverläufe und Stärken der von außen einwirkenden Felder. Beispielsweise benötigen sensorische Nervenfasern, die auch für die Weiterleitung von Schmerzsignalen zuständig sind, wegen ihres geringeren Durchmessers eine höhere Feldstärke zur Depolarisation (d.h. zur Auslösung eines Aktionspotentials) als motorische Nervenfasern. Somit ist es möglich, bei moderaten Reizstärken nur die motorischen, nicht aber die sensorischen Fasern anzuregen und somit nahezu schmerzfrei Nerven zu reizen.

Insbesondere kann für diese Art der Reizung auch das Prinzip der magnetischen Induktion eingesetzt werden. Dabei erzeugt ein zeitlich veränderliches magnetisches Feld ein induziertes elektrisches Feld. Das zeitlich veränderliche Magnetfeld kann durch eine Spule erzeugt werden, welche von einem zeitlich veränderlichen Strom durchflossen wird. Diese Spule, liegt beispielsweise auf der Haut über dem zu reizenden Nervengewebe auf, so dass das erzeugte Magnetfeld das Gewebe durchdringen kann und nach dem Induktionsprinzip die für die Reizung erforderlichen Ströme im Gewebe erzeugt. Die Reizung durch diese sogenannte induktive Magnetstimulation kann dabei berührungslos erfolgen, da das Magnetfeld Körpergewebe ungehindert durchdringen kann. Die zeitabhängigen Magnetfelder werden über kurze Strompulse einer Dauer von üblicherweise 50 - 400 Mikrosekunden erzeugt. Das Prinzip der induktiven Reizung basiert grundsätzlich auf einer zeitlichen Änderung des magnetischen Feldes. Somit können auch nur zeitlich veränderliche elektrische Felder im Gewebe entstehen. Daher können hier beispielsweise keine effizienten einfachen monophasischen Rechteckpulse mit Gleichanteil erzeugt werden, wie sie bei der Elektrostimulation eingesetzt werden.

Ein Vorteil der induktiven Magnetstimulation liegt in der Berührungslosigkeit, da das Magnetfeld der Spule Körpergewebe auch in einem gewissen Abstand zur Spule erreicht. Daher können Nervenzellen auch steril gereizt werden. Ein weiterer Vorteil ist, dass das Verfahren im Gegensatz zur elektrischen Stimulation über Elektroden nahezu völlig schmerzfrei ist, da anders als bei der Elektrostimulation keine hohen Stromdichten an Einspeiseorten von Elektroden entstehen können. Aus diesen Gründen eignet sich das Verfahren auch besonders gut zur Reizung tief liegender Gewebestrukturen (z.B. die Gehirnrinde durch den Schädelknochen hindurch) und zur schmerzfreien Muskelstimulation z.B. im Bereich der Rehabilitation.

Infolge dieser Vorteile konnte sich die induktive Magnetstimulation gegenüber der Elektrostimulation bereits in einigen Bereichen durchsetzen oder sogar neue Anwendungsgebiete erschließen. Sehr verbreitet ist das Verfahren zur Anwendung auf das zentrale sowie das periphere Nervensystem.

Momentan handelt es sich um das einzige nicht-invasive Verfahren, mit dem ohne Schmerzen der Person beispielsweise bestimmte Gehirnbereiche gezielt in der Weise angesprochen werden können, (d.h. Auslösen von Nervenaktionspotentialen oder unterschwellige Beeinflussung von Nervenzellen in diesen Bereichen), dass Reaktionen der Nervenzellen vom Körper genauso oder zumindest sehr ähnlich wie natürlich entstandene Nervenimpulse verarbeitet werden.

Die induktive Magnetstimulation wird in der Grundlagenforschung als Instrument zur gemeinsamen Untersuchung zusammen mit der funktionellen Magnetresonanztomographie eingesetzt. Über Pulse ist eine gezielte Anregung (und Hemmung) bestimmter Hirnareale erzeugbar, deren Auswirkungen wiederum mit der Magnetresonanztomographie untersucht werden können.

Weiterhin bestehen Anwendungen der induktiven Magnetstimulation hinsichtlich peripherer motorischer Nerven. Hierbei ist besonders die repetitive Dauerstimulation mit schnellen Pulsfolgen (ca. 10 bis 50 Pulse pro Sekunde) von großer Bedeutung.

Darüber sind Anwendungen bei im Hochleistungssport eingesetzten Geräten bekannt.

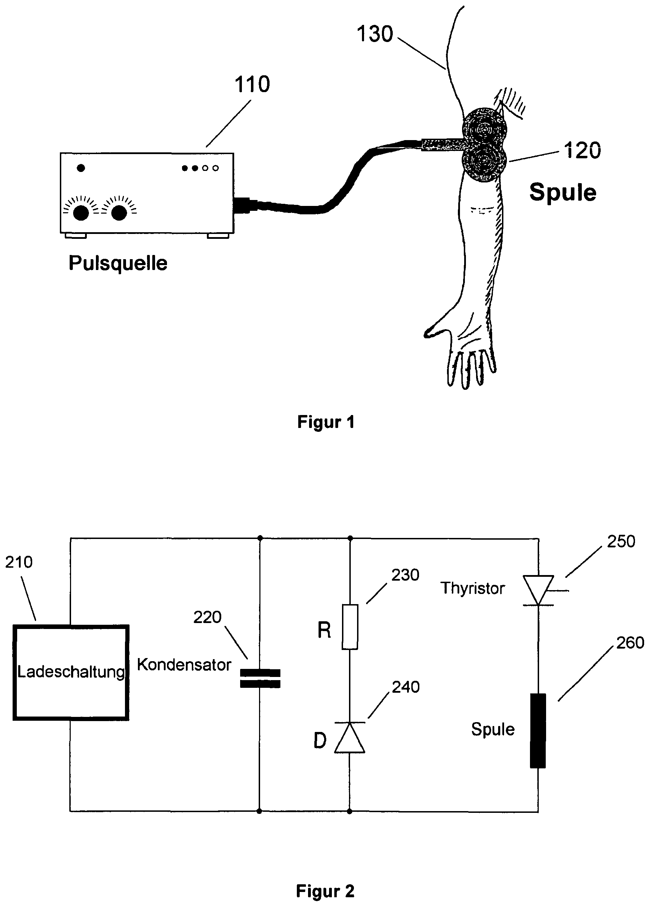

Figur 1 zeigt eine typische Anordnung des bisherigen Einsatzes der induktiven Magnetstimulation. Die Pulsquelle 110 erzeugt einen kurzen starken Strompuls und leitet diesen an die Spule 120. Die Spule 120 wird nahe des zu reizenden Nervengewebes des Körpers positioniert, so dass das erzeugte Magnetfeld diese Gewebestruktur durchdringen kann. Das von der Spule erzeugte magnetische Feld induziert im Körpergewebe, hier am Oberarm 130, ein elektrisches Feld, welches über die entstehenden Ströme Nerven- und Muskelgewebe reizt.

Allerdings hat bei der induktiven Magnetstimulation dieser Umweg über das Magnetfeld der Spule auch wichtige technische Probleme zur Folge:

Die erforderlichen magnetischen Flussdichten liegen im Bereich von ca. 1 Tesla, so dass während des sehr kurzen magnetischen Stimulationspulses eine extrem hohe elektrische Leistung in die Spule hineingeführt werden muss, um die entsprechenden Feldenergien zu erzeugen. Die erforderlichen elektrischen Leistungen können Werte von mehreren Megawatt und die Ströme mehrere Kiloampere bei Spannungen von einigen Kilovolt erreichen. Daher sind die Pulsquellen technisch aufwändig; weiterhin überhitzt die Spule durch die Stromwärmeverluste sehr schnell, wobei hier zusätzlich beachtet werden muss, dass die Spule als Teil, welches den Körper direkt berühren kann, keinesfalls zu hohe Temperaturen erreichen darf.

Um dennoch mit vertretbarem technischen Aufwand entsprechende Ströme und Energien für diese Art der Reizung zur Verfügung stellen zu können, arbeiten Magnetstimulationsgeräte derzeit nach dem Prinzip des resonanten Schwingkreises, bei dem ein Kondensator seine Energie in die Spule entlädt. Das Prinzip zur Erzeugung leistungsstarker Pulse für die Spule beruht somit auf einer kontinuierlichen Ladung des Schwingkreis-Kondensators über eine Ladevorrichtung bei relativ geringer Leistung und der schnellen Abgabe des Energieinhalts dieses Kondensators an die Spule zur Erzeugung des kurzen starken Magnetfeld-Pulses.

Figur 2 zeigt den prinzipiellen Schaltungsaufbau eines induktiven Stimulationsgerätes, wie er in den ersten Geräten insbesondere zur kontaktlosen Reizung von kortikalen Nervenstrukturen durch den intakten Schädelknochen verwendet wurde (R. Siebner, U. Ziemann, "Das TMS-Buch", Springer Verlag, ISBN-13 978-3-540-71904-5). Die Schaltung nutzt hierzu einen leistungsstarken gedämpften elektrischen Schwingkreis (Resonator) bestehend aus einem Kondensator 220, einem Dämpfungswiderstand 230, einer Diode 240, einem Thyristor 250 und der Spule 260. Die Ladeschaltung 210 lädt den Kondensator 220 auf eine Spannung von mehreren tausend Volt auf. Der Energieinhalt des Kondensators beträgt dabei einige 100 Joule. Der Thyristor 250 dient als Schalter, der beim Zünden den Kondensator 220 mit der magnetischen Spule 260 verbindet und so den Stromfluß in der Spule beginnen läßt.

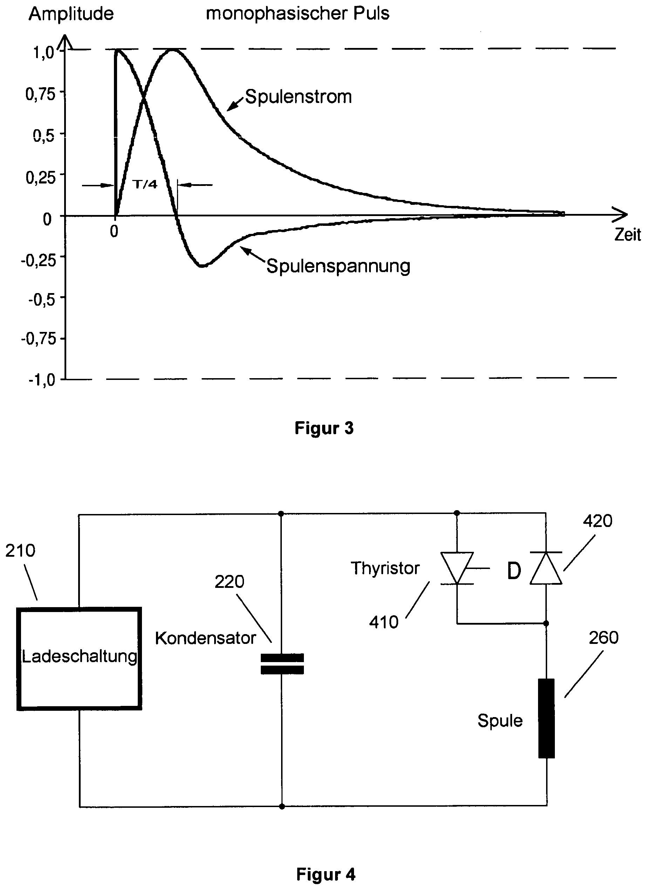

Figur 3 zeigt den zeitlichen Verlauf von Strom und Spannung in der Spule entsprechend der Schaltung von Figur 2. Nach dem Zünden des Thyristors entsteht ein zunächst sinusförmig ansteigender Stromfluss, der ein entsprechend mit der Zeit zunehmendes Magnetfeld erzeugt. Dieses magnetische Feld induziert wiederum infolge seiner zeitlichen Änderung Ringströme im Körpergewebe. Bei Erreichen des Strom-Scheitelwertes hat die phasenversetzte Spulenspannung genau ihren ersten Nulldurchgang. Da ab diesem Zeitpunkt die Spulenspannung ihr Vorzeichen umkehrt, wird nun die Dämpfungsschaltung, bestehend aus dem Widerstand 230 und der Diode 240 aktiv, welche ein Weiterschwingen des Schwingkreises unterbindet. Daher sinkt der Spulenstrom nach Erreichen seines Scheitelwertes langsam wieder auf Null zurück. Die typische Zeitdauer zwischen der Thyristorzündung und dem Erreichen des Stromscheitelwertes liegt bei ca. 50 bis 150 Mikrosekunden. Durch diese Dämpfungsschaltung wird allerdings die gesamte Pulsenergie des Kondensators im Widerstand 230 und in den Spulenleitern der Spule in Wärme umgewandelt.

Diese bei den ersten Geräten eingesetzte Dämpfungsschaltung, welche die Schwingung ab der ersten abfallenden Stromflanke (nach einem Viertel der Periodendauer) abdämpft, charakterisiert die sogenannte monophasische Stimulation, da der Spulenstrom während des Pulses nur in eine Richtung fließt, also sein Vorzeichen nicht wechselt. Da bei diese Geräten die Pulsenergie des magnetischen Feldes bei jedem Puls komplett verloren geht, weisen diese Geräte einen besonders hohen Energieverbrauch auf.

Diese ersten Geräte waren daher nicht für die sogenannte repetitive Stimulation geeignet, bei der 10 bis 50 Pulse pro Sekunde erforderlich sind. Weiterhin erschweren aber auch die Größe der Geräte und ihr hoher Preis das Erschließen weiterer Einsatzgebiete.

Daher besteht das wichtigste Entwicklungsziel bei den Geräten zur induktiven Magnetstimulation in der Reduktion des Energieverbrauches und der Erwärmung der Spule (R. Siebner, U. Ziemann, "Das TMS-Buch", Springer Verlag, ISBN-13 978-3-540-71904-5). Durch experimentelle Untersuchungen konnte gezeigt werden, dass auch ein ungedämpfter sinusförmiger Zeitverlauf des Spulenstromes und damit auch des magnetischen Feldes bei gleicher Amplitude etwa eine gleichwertige Wirkung hinsichtlich der Nervenreizung zeigt, wie der Stromverlauf von Figur 3.

Figur 4 zeigt den prinzipiellen Schaltungsaufbau eines weiteren bekannten Stimulationsgerätes, wie er in einer späteren Gerätegeneration verwendet wurde. Dieses Gerät erzeugt sinusförmige Strom- bzw. Feldpulse. Auch hier lädt die Ladeschaltung 210 den Kondensator 220 auf eine Spannung von mehreren tausend Volt auf. Der Thyristor 410 dient wieder als Schalter, der beim Zünden den Kondensator 220 mit der magnetischen Spule 260 verbindet. Im Gegensatz zur monophasischen Stimulatorschaltung von Figur 2 wird bei dieser Schaltung jedoch keine Dämpfungsschaltung eingesetzt, so dass auch nach dem ersten Nulldurchgang der Spulenspannung der Schwingkreis weiterschwingt.

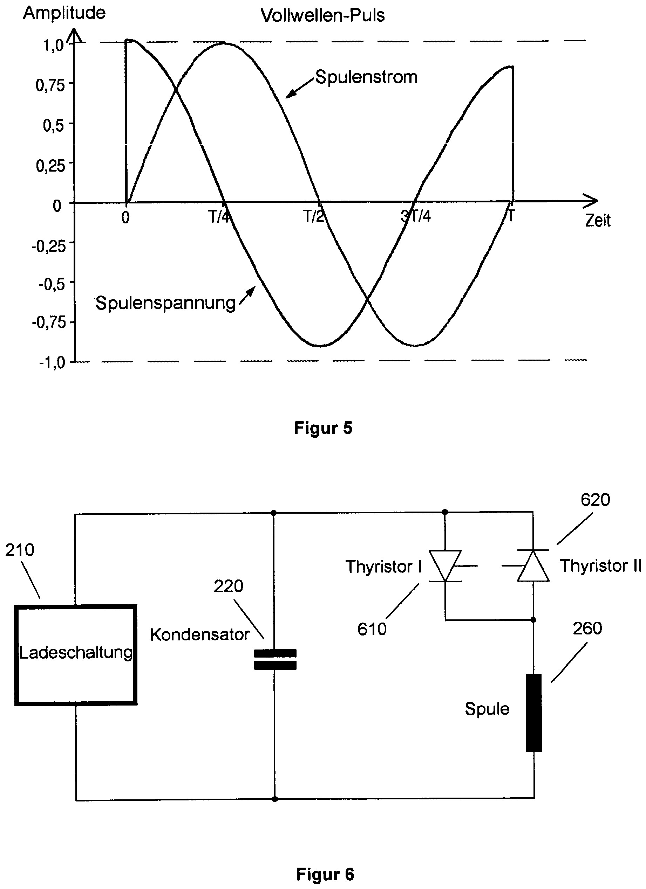

Figur 5 zeigt den zeitlichen Verlauf von Strom und Spannung in der Spule entsprechend der Schaltung von Figur 4. Nach dem Zünden des Thyristors entsteht ein sinusförmig ansteigender Stromfluss, der ein entsprechend mit der Zeit zunehmendes Magnetfeld erzeugt. Nach einer halben Sinusschwingung, zum Zeitpunkt T/2 wechselt der Strom im Schwingkreis seine Polarität. Zu diesem Zeitpunkt übernimmt die Diode 420 die Leitung des Spulenstromes bis eine volle Sinusschwingung zum Zeitpunkt T erreicht wird. Eine erneute Umkehr der Stromrichtung und damit ein Weiterschwingen wird unterbunden, da der Thyristor 410 zu diesem Zeitpunkt T nicht mehr leitet. Wegen der Umkehr der Stromrichtung während eines Pulses zum Zeitpunkt T/2 wird diese Art der Stimulation allgemein als biphasische Magnetstimulation bezeichnet.

Durch das Schaltungsprinzip gemäß Figur 4 kann erreicht werden, dass ein großer Teil der für die Spule 260 aufgewendeten Feldenergie wieder in den Kondensator 220 zurückgeführt werden kann und so die Verluste sowohl in der Pulsquelle als auch in der Spule 260 reduziert werden. Die Verluste der Schaltung nach Figur 4 ergeben sich hauptsächlich über die ohmschen Widerstände der beteiligten Schaltungskomponenten und ihrer Verbindungskabel.

Da die für eine erfolgreiche Reizung erforderliche Stromamplitude jedoch gegenüber den Geräten mit monophasischer Pulsform etwa unverändert ist, bleiben auch die nötige Spannung und der Energieinhalt des Kondensators 220 nahezu gleich, wie bei monophasischen Geräten.

Figur 6 stellt eine weitere bekannte Entwicklung der Schaltkreise von induktiven Magnetstimulatoren dar, wie er in einer späteren Gerätegeneration verwendet wurde (R. Siebner, U. Ziemann, "Das TMS-Buch", Springer Verlag, ISBN-13 978-3-540-71904-5). Auch hier lädt die Ladeschaltung 210 den Kondensator 220 auf eine Spannung von mehreren tausend Volt auf. Der Thyristor 610 dient wieder als Schalter, der beim Zünden den Kondensator 220 mit der magnetischen Spule 260 verbindet.

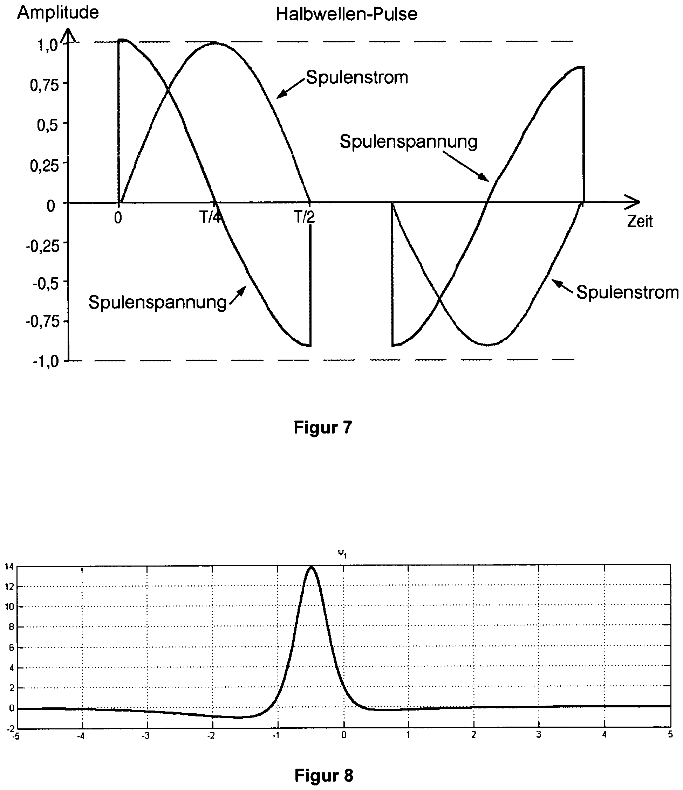

Figur 7 zeigt den zeitlichen Verlauf von Strom und Spannung in der Spule entsprechend der Schaltung von Figur 6. Nach dem Zünden des Thyristors 610 entsteht ein sinusförmig ansteigender Stromfluss, der ein entsprechend mit der Zeit zunehmendes Magnetfeld erzeugt. Nach einer halben Sinusschwingung, zum Zeitpunkt T/2, erreicht der Strom im Schwingkreis seine erste Nullstelle. Falls zu diesem Zeitpunkt der zweite Thyristor 620 nicht gezündet wird, ist eine Umkehr der Stromrichtung nicht möglich, so dass ein Weiterschwingen bereits nach einer Halbwelle unterbunden wird. Eine Zündung des Thyristors 620 zu einem späteren Zeitpunkt erzeugt in der Spule einen weiteren Halbwellenpuls mit umgekehrter Strom- und Magnetfeldrichtung. Alternativ kann aber auch direkt bei Erreichen der ersten Strom-Nullstelle der zweite Thyristor 620 gezündet werden, so dass eine volle Sinusschwingung, ähnlich wie bei Figur 5, entsteht. In jedem Fall wird aber auch bei dieser Schaltung die Feldenergie der Spule zu einem großen Teil wieder in den Kondensator zurückgeführt.

Je nach Wahl des Endzeitpunktes des Pulses unterscheidet man daher bezüglich der Pulsformen der induktiven Stimulationsgeräte zwischen einer biphasischen Vollwellen-Stimulation (Dauer des Strompulses eine volle Sinusperiode) und einer biphasischen Halbwellen-Stimulation. Nachteilig bei der biphasischen Halbwellen-Stimulation ist allerdings, dass nach dem Puls die Spannungsrichtung im Kondensator gegenüber dem Zustand vor der Pulsabgabe invertiert ist, wodurch die entsprechende Ladeschaltung aufwändiger wird. Weiterhin wechselt bei der biphasischen Halbwellenstimulation auch die Richtung des magnetischen Feldes, so dass aufeinanderfolgende Pulse leicht unterschiedliche Wirkungen im Gewebe erzeugen.

Die Energierückgewinnung entsprechend den Schaltungen von Figur 4 und Figur 6 ermöglicht eine Reduktion der bei jedem Puls verlorenen Energie und damit auch der Stromwärmeverluste in Spule und Leistungselektronik. Dies ermöglicht auch den Bau von repetitiven induktiven Stimulationsgeräten, welche bis zu 100 Pulse pro Sekunde abgeben können. Allerdings ist speziell für diesen repetitiven Betrieb der Energieverbrauch und die Spulenerwärmung immer noch erheblich. Insbesondere die Spulenerwärmung begründet sich durch die sehr hohen notwendigen Spulenströme im Kiloampere-Bereich.

Eine weitere Möglichkeit zur Reduktion der Energieverluste kann über die Senkung der Stromwärmeverluste der Spule erreicht werden (R. Siebner, U. Ziemann, "Das TMS-Buch", Springer Verlag, ISBN-13 978-3-540-71904-5). Dies geschieht durch Erhöhung des wirksamen Leiterquerschnitts, wobei einerseits ein dickeres Leitermaterial verwendet werden kann und andererseits der Leiter durch Verwendung von Hochfrequenzlitze filamentiert werden kann, so dass die Stromverdrängungseffekte im Leiter reduziert werden. Allerdings ist der elektrische Widerstand der Spule aus Gewichtsgründen nicht beliebig reduzierbar

Hinsichtlich des zeitlichen Verlaufs des Reizpulses stellen noch immer die drei genannten Wellentypen, die gedämpften monophasischen Pulse, die biphasische Halbwellen-Pulse und die biphasischen Vollwellen-Pulse die einzigen Pulsformen dar, die bei kommerziellen induktiven Magnetstimulationsgeräten eingesetzt werden. Alle diese Wellenformen basieren letztlich auf dem Prinzip des resonanten Schwingkreises, bei dem die Spule die Induktivität darstellt.

Daher haben die bisher eingesetzten Geräte zusätzlich den großen Nachteil, dass die Pulsdauer von der Induktivität der Spule abhängt. Insbesondere weisen beispielsweise kleine Spulen oft konstruktionsbedingt eine verringerte Induktivität als große Spulen auf; daher konnte bei bisherigen Systemen die Pulsdauer bei Einsatz verschiedener Spulen nicht konstant in einem optimalen Bereich gehalten werden.

Zeitweise Versuche mit anderen Pulsformen, wie in Peterchev et al. 2008 mit einer Rechteckform (A. V. Peterchev, R. Jalinous, and S. H. Lisanby: A Transcranial Magnetic Stimulator Inducing Near-Rectangular Pulses With Controllable Pulse Width (cTMS), IEEE Transactions on Biomedical Engineering, vol. 55, no. 1, 2008) sind entweder energetisch sehr ineffektiv, oder sie führen zu äußerst aufwändigen technischen Aufbauten und sind somit zu kostspielig für eine kommerzielle technische Realisierung.

WO 2007/145838 A2 offenbart eine Vorrichtung und ein Verfahren zum bereitstellen steuerbarer Pulsparameter einer magnetischen Stimulation. In bestimmten Ausführungsformen enthält die Vorrichtung 2 Kondensatoren, die mit einer Ladeschaltung verbunden sind. Die Kondensatoren können abwechselnd über jeweilige Halbleiterschalter mit der Stimulationsspule so verbunden werden, dass die entstehenden Spulenströme ein induziertes elektrisches Feld mit nahezu rechteckförmigem Verlauf zur Folge haben. Die Kondensatorspannungen und die Zeitpunkte zu denen die Halbleiterschalter geschaltet werden, können dabei gesteuert werden.

US 2009/018384 A1 beschreibt ein modulares Magnetstimulationsgerät. Wesentliche Komponenten des Gerätes sind eine Batterie zur Stromversorgung, eine Stromversorgungsschaltung, ein Energiespeicher in Form eines Kondensators, eine Steuereinheit, eine Ladungs-Rückspeiseeinheit und eine Stimulationsspule. Über Halbleiterschalter kann die Spule mit einem Kondensator verbunden werden und so einen Stimulationspuls erzeugen.

EP 1 894 600 A1 offenbart eine magnetische Stimulationsvorrichtung zur magnetischen Stimulation des menschlichen Gehirns. Zwei runde Spulen werden dabei von der Vorrichtung entweder mit gegenläufigen oder gleichsinnigen Strömen angesteuert, so dass ein Gradientenfeld oder ein gleichmäßiges Magnetfeld entsteht.

GOETZ S M ET AL: "Analysis of a novel magnetic stimulation system: Magnetic harmonic multicycle stimulation (MHMS)", BIOMEDICAL AND PHARMACEUTICAL ENGINEERING, 2009. ICBPE '09. INTERNATIONAL CONFERENCE ON, IEEE, PISCATAWAY, NJ, USA, 2. December 2009, Seiten 1-6 untersucht den Einfluss von Feldpulsen mit mehrfacher gedämpfter Sinus-Schwingungsform auf die Reizschwelle bei magnetischer Nervenstimulation.

DE 35 24 232 A1 offenbart ein universelles Elektrotherapiegerät zur Erzeugung unterschiedlicher Reizströme, Interferenzströme oder Magnetfelder über Elektroden oder Magnetspulen.

Bei allen Anwendungen besteht der Nachteil der induktiven Magnetstimulation daher immer noch im hohen Energieverbrauch, der sehr schnellen Überhitzung der Spule und dem hohen Gewicht der Lade- und Pulserzeugungselektronik.

Ein weiterer Nachteil besteht darin, dass der zeitliche Verlauf des Reizpulses nicht flexibel an bestimmte Nervenzell- oder Axontypen oder an andere Erfordernisse individuell angepasst werden kann.

## ÜBERSICHT ÜBER DIE ERFINDUNG

Es ist Aufgabe der Erfindung, eine Vorrichtung zur Erzeugung frei wählbarer oder optimierter magnetischer Pulse zur Reizung von Nerven- und/oder Muskelzellen bereitzustellen, mit dessen Hilfe die genannten Nachteile vermieden werden.

Diese Aufgabe wird gelöst durch eine Vorrichtung zur Erzeugung von magnetischen Feldpulsen über Strompulse in einer Spule mit den im Anspruch 1 genannten Merkmalen. Vorteilhafte Ausgestaltungen der Erfindung sind jeweils Gegenstand der Unteransprüche.

Die Erfindung basiert einerseits auf der Erkenntnis, dass bei einer verbesserten Anpassung des Zeitverlaufes der im Gewebe induzierten Felder und Ströme an die dynamischen Ladungstransportphänomene der Nerven- oder Muskelfasern die benötigte Feldstärke und Feldenergie zur induktiven Reizung dieser Fasern reduziert werden kann. Andererseits liegt der Erfindung die Erkenntnis zu Grunde, dass der Zeitverlauf des Stimulationspulses in einem weiten Bereich anpassbar sein muss um entweder unterschiedliche Zelltypen optimal reizen zu können oder unterschiedliche Optimierungskriterien zu erfüllen. Insbesondere ist hierzu von der zugehörigen Leistungselektronik der zeitliche Verlauf des kurzen Magnetfeldpulses den die Spule erzeugt, gegenüber bisherigen Systemen in der Weise zu verändern, dass nicht-sinusförmige Pulse mit Verlaufsformen erzeugt werden, wie sie entweder als optimiertes Ergebnis einer numerischen Simulation des Membranverhaltens oder als Resultat einer optimierenden experimentellen Untersuchung an Nervenzellen ergeben.

Bevorzugt werden magnetische Feldpulse mit wählbarem Zeitverlauf erzeugt, die im Körpergewebe elektrische Reizströme hervorrufen und dadurch wiederum Aktionspotentiale von Nerven- und/oder Muskelzellen auslösen. Der zur Erzeugung der Feldpulse über eine Spule notwendige Strom sollte dabei unter mehreren möglichen Formen wählbar sein. Insbesondere sollten diese möglichen Formen für die Vorrichtung von aussen vorgegeben werden können, z.B. in Form eines Datensatzes. Vorzugsweise sollten hier Pulsformen vorgegeben werden können, deren zeitliche Verlaufsformen hinsichtlich bestimmter Parameter optimiert sind.

In einer anderen Variante wird der zeitliche Verlauf des Strompulses über ein Rechenverfahren berechnet, welches numerisch das elektrische Verhalten der Nerven- und/oder Muskelzellen und der Spule nachbildet und den zeitlichen Verlauf des Strompulses hinsichtlich mindestens eines Parameters optimiert.

In einer weiteren Variante wird der zeitlichen Verlauf des Strompulses über die Reizung der Nerven- und/oder Muskelzellen mit Strompulsen vorgegebener Form ermittelt, wobei auch hier ein Optimieren des zeitlichen Verlaufs des Strompulses hinsichtlich mindestens eines Parameters erfolgt. Weiterhin werden dabei wesentliche Kenngrößen der gereizten Nerven-und/oder Muskelzellen aus diesem optimierten Strompuls ermittelt.

Beide genannten Varianten können für das Bestimmen des zeitlichen Verlaufs des Strompulses über die Simulation oder Reizung der Nerven- und/oder Muskelzellen als Strompulse vorgegebener Form Rauschsignale verwendet werden und diese Rauschsignale zeitlich auf die ausgelösten Aktionspotentiale der Nerven- und/oder Muskelzellen synchronisiert werden. Diese genannte Optimierung der Verlaufsform kann sich auf verschiedene technisch-physikalische Größen beziehen, die erforderlich sind um eine Reizung auszulösen; beispielsweise kann sich diese Optimierung auf eine Minimierung der benötigten Feldenergie, der Spulenverlustenergie, der erforderlichen Spulenstromstärke, der erforderlichen Spulenspannung, die maximale Steilheit der Spulenspannung oder des Spulenstromes oder auf den akustischen Artefakt der Spule beziehen.

Mit anderen Worten sollen die von der Leistungselektronik erfindungsgemäß produzierten Strompulse für die Spule nicht mehr einen sinusförmigen oder gedämpft sinusförmigen Verlauf aufweisen, sondern so beschaffen sein, dass das von der Spule induzierte elektrische Feld einen zeitlichen Verlauf aufweist, der bei gleicher Reizwirkung eines der genannten Optimierungskriterien erfüllt.

Die Ausführungsformen der Erfindung unterscheiden sich daher von Schaltungen, die zusammen mit der Spule und einem Kondensator einen gedämpften Schwingkreis bilden, dessen Stromverlauf sich aus Teilen von gedämpften Sinuswellen zusammensetzen, deren Übergänge zwischen diesen Teilen jeweils entweder bei Nulldurchgängen oder bei Erreichen von Maxima und Minima des Stromes durch die Spule erfolgen.

Insbesondere lassen sich die Stromwärmeverluste $E_v$ dabei über das zeitliche Integral des Quadrates des Spulenstromes $I_{sp}$ über die Pulsdauer $\tau$ multipliziert mit dem Innenwiderstand $R_i$ der Spule abschätzen:

$$E_v = \int I_{sp}^2 \cdot R_i \cdot dt$$

Die erforderliche maximale Stärke des magnetischen Feldes lässt sich - bei unveränderter Spule - über den maximalen Spulenstrom abschätzen.

Weiterhin sollte die Leistungselektronik zur Erzeugung der Strompulse in der Spule vorzugsweise so ausgeführt werden, dass die hohen erforderlichen elektrischen Leistungen für die Erzeugung des magnetischen Feldes der Reizspule aus Energiespeichern (z.B. Kondensatoren) bezogen werden können, um eine gleichmäßige Belastung des Stromnetzes zu erreichen und dass weiterhin der größte Teil dieser Feldenergie der Spule wieder in diese Energiespeicher zurückgeführt werden kann.

Vorzugsweise kann diese Leistungselektronik dabei so ausgeführt sein, dass die Spule über ein pulsweitenmoduliertes Signal (PWM-Signal) eines Stromrichters angesteuert wird, wobei die einzelnen Pulse dieses PWM-Signals wesentlich kürzer als der gesamte zur Reizung der Nerven verwendete Puls sein sollten, so dass der Spulenstrom über die Pulsweite dieses PWM-Signals gesteuert werden kann. Über die Steuerung des zeitlichen Verlaufs des Spulenstroms kann entsprechend das im Körpergewebe induzierte elektrische Feld gesteuert werden. Dadurch kann der Zeitverlauf des im Gewebe induzierten Feldes an die dynamischen Ladungstransportphänomene der Nervenfasern so angepasst werden, dass die genannten Optimierungskriterien erfüllt werden.

Als Stromrichter wird erfindungsgemäß das Konzept des modularen Multilevel Konverters eingesetzt .

Dieser Stromrichter wird aus mehreren unabhängig voneinander steuerbaren Einzelmodulen aufgebaut, welche jeweils aus Leistungs-MOSFETs und einem eigenen SpeicherKondensator aufgebaut sind. Die für die Erzeugung erforderliche Feldenergie der Spule kann daher aus allen Kondensatoren dieser Module entnommen werden. Weiterhin kann die Feldenergie der Spule entsprechend auch wieder in die Kondensatoren rückgespeist werden. Jedes Einzelmodul besitzt zwei Leistungsanschlüsse und kann jeweils in beiden Polarisationsrichtungen im PWM-Betrieb geladen und entladen werden.

Ein Vorteil der Verwendung des modularen Multilevel Konverters liegt darin, dass es durch geeignete Verschaltung des gesamten Stromrichters möglich ist, mit niedrigen Versorgungsspannungen der Einzelmodule hohe Pulsspannungen an der Stimulationsspule abzugeben.

Ein weiterer Vorteil der Verwendung des modularen Multilevel Konverters liegt darin, dass die Spulenspannung nicht nur über das Tastverhältnis des PWM-Signals sondern auch über die Spannung und Anzahl der momentan aktiven Einzelmodule gesteuert werden kann.

Ein weiterer Vorteil der Verwendung des modularen Multilevel Konverters liegt darin, dass durch asynchrones Steuern der Einzelmodule das Ausgangssignal des Stromrichters eine deutlich feinere zeitliche Stufung des PWM-Signals aufweist als die zeitliche Stufung eines einzelnen Moduls oder eines alternativ verwendeten Stromrichters.

Insbesondere kann bei Realisierung des modularen Multilevel Konverters als Stromrichter für die Erzeugung der Spulenpulse durch Verwendung vieler seriell geschalteter Module die Spannung an den einzelnen Modulen noch weiter reduziert werden. Daher können in diesen Modulen elektronische Bauelemente (z.B. Leistungstransistoren und Speicherkondensatoren) mit relativ geringer Spannungs-festigkeit eingesetzt werden.

Weiterhin kann vorzugsweise zwischen die Leistungselektronik und die Spule noch eine geeignete Glättungsschaltung eingefügt werden, die den Verlauf der Spulenspannung und des Spulenstromes gegenüber dem PWM-Signal glättet.

Ein Vorteil der vorliegenden Erfindung besteht in der Bereitstellung einer Vorrichtung zur induktiven Nervenreizung, welche eine vergleichsweise geringe Feldenergie und Feldstärke zur Reizung der Nerven benötigen. Mit dieser Reduktion der Energie kann auch der eine oder mehrere für die Zwischenspeicherung der Pulsenergie genutzten Kondensatoren hinsichtlich ihrer Baugrößen reduziert werden.

Ein weiterer Vorteil der vorliegenden Erfindung besteht in der Bereitstellung einer Vorrichtung zur induktiven Nervenreizung, welche durch die Reduktion der nötigen Feldenergie eine vergleichsweise geringe Spulenspannung für die Reizung der Nerven benötigt, so dass Isolationsabstände im Leistungskreis verkleinert und notwendige Sicherheitsmaßnahmen vereinfacht werden können.

Unter Leistungskreis wird dabei der elektrische Schaltungsteil einer Vorrichtung zur Erzeugung von Magnetfeldpulsen verstanden, in welchem die für den Puls erforderlichen Ströme und Spannungen erzeugt werden.

Ein weiterer Vorteil der vorliegenden Erfindung besteht in der Bereitstellung einer Vorrichtung zur induktiven Nervenreizung, welche einen vergleichsweise geringen Spulenstrom für die Reizauslösung benötigt. Auf diese Weise können einerseits die Stromwärme-Verluste in der Spule und im Zuführungskabel reduziert werden, andererseits können im zur Pulserzeugung verwendeten Leistungskreis elektronische Leistungsbauteile von vergleichsweise geringerer Stromtragfähigkeit und entsprechend geringerer Baugröße eingesetzt werden.

Ein weiterer Vorteil der vorliegenden Erfindung besteht in der Bereitstellung einer Vorrichtung zur induktiven Nervenreizung, welche sich auf Grund ihres vergleichsweise geringen Energieverbrauchs vor allem für die repetitive Stimulation mit Pulswiederholraten von 10 bis 1000 Pulsen pro Sekunde eignet. Gleichzeitig können die Geräte zur erfindungsgemäßen Pulserzeugung vergleichsweise klein, leicht und damit tragbar gemacht werden, so dass sie sich auch für mobile Anwendungen oder den Home-Care Bereich eignen.

Ein anderer Vorteil der vorliegenden Erfindung besteht in der Bereitstellung einer Vorrichtung zur effizienten Erzeugung von nahezu beliebigen, frei wählbaren Zeitverläufen der Magnetfeldpulse bei gleichzeitig hoher Leistung und geringen Verlusten. Insbesondere können die Pulse so geformt werden, dass die Zeitverläufe der im Gewebe induzierten Ströme an die dynamischen Ladungstransportphänomene der Nervenfasern optimal angepasst sind und damit die in der Spule zur Reizung benötigte Feldstärke und Feldenergie reduziert werden kann.

Ein weiterer Vorteil der vorliegenden Erfindung besteht darin, dass die Verwendung eines Leistungskreises zur Erzeugung frei wählbarer Stromverläufe für den Spulenspuls es auch ermöglicht, das vom selben Gerät wahlweise unterschiedlich geformte Pulse abgegeben werden können. Somit können einerseits Pulse generiert werden, die bevorzugt ganz bestimmte Typen von Nervenzellen reizen können. Weiterhin können die Pulse aber auch so geformt sein, dass sie eine geringe Geräuschentwicklung in der Spule zur Folge haben oder dass sie eine maximale Reizwirkung unter Vernachlässigung der Spulenverluste aufweisen.

KURZBESCHREIBUNG DER ZEICHNUNGEN

Die voranstehenden und weitere Ziele, Merkmale und Vorteile der vorliegenden Erfindung werden aus der folgenden, detaillierten Beschreibung in Zusammenhang mit den beigefügten Zeichnungen deutlicher, wobei:

Fig. 1     eine Pulsquelle, die über ein Kabel angekoppelte Spule und die zu reizende Gewebsstruktur (menschlicher Oberarm) zeigt;

Fig. 2     den prinzipiellen Aufbau eines monophasischen Leistungskreises zeigt;

Fig. 3     den Spannungs- und Stromverlauf in der Spule eines monophasischen Stimulators während eines Pulses zeigt;

Fig. 4     den prinzipiellen Aufbau eines Leistungskreises zur Erzeugung von Sinus-Vollwellen zeigt;

Fig. 5     den Spannungs- und Stromverlauf eines Vollwellen-Stimulators in der Spule während eines Pulses zeigt;

Fig. 6     den prinzipiellen Aufbau eines Leistungskreises zur Erzeugung von Sinus-Halbwellen zeigt;

Fig. 7     den Spannungs- und Stromverlauf eines Halbwellen-Stimulators in der Spule während eines Pulses zeigt;

Fig. 8     beispielhaft einen optimierten Zeitverlauf des erforderlichen Stromes an einer Zellmembran (und damit auch des Verlaufes der Spulenspannung) während eines Reizpulses, wie er sich als optimiertes Resultat einer Nervenzellmodellierung ergibt;

Fig. 9      eine Strompulsform zeigt, die sich aus zwei Sinus-Halbwellen unterschiedlicher Frequenzen zusammensetzt;

Fig. 10     die Veränderung der Auslöseschwelle für eine Nervenfaser in Abhängigkeit des Quotienten der Frequenzen von zwei aneinandergereihten Halbwellen unterschiedlicher Frequenz gemäß Figur 9 zeigt;

Fig. 11     beispielhaft ein Blockschaltbild, wie es zur Simulation einer Stimulationsumgebung zur Ermittlung optimaler Pulsformen eingesetzt werden kann zeigt;

Fig. 12     beispielhaft Ausgangssignale eines PWM-Stromrichters bei verschiedenen Tastverhältnissen zeigt;

Fig. 13     beispielhaft zwei Varianten eines Halbbrückenmoduls eines Modularen Multilevel Konverters zeigt;

Fig. 14     beispielhaft ein einzelnes Vollbrückenmodul eines Modularen Multilevel Konverters zeigt;

Fig. 15     beispielhaft eine Ausführungsform zur Verwendung eines Modularen Multilevel Konverters zur bipolaren Speisung der Stimulationsspule zeigt;

Fig. 16     beispielhaft eine weitere Ausführungsform zur Verwendung eines Modularen Multilevel Konverters zur bipolaren Speisung der Behandungsspule zeigt;

Fig. 17     beispielhaft PWM-Ausgangssignale eines Modularen Multilevel Konverters mit 3 Spannungsniveaus bei verschiedenen Tastverhältnissen zeigt; und

Fig. 18     beispielhaft eine Glättungsschaltung zeigt, wie sie zwischen den Ausgang eines Stromrichters und die Stimulationsspule eingefügt werden kann, um den Verlauf der Spulenspannung und des Spulenstromes gegenüber dem PWM-Signal zu glätten.

In den Zeichnungen sollen gleiche Bezugszeichen gleiche Teile, Bauteile und Anordnungen bezeichnen.

DETAILLIERTE BESCHREIBUNG BEVORZUGTER AUSFÜHRUNGSFORMEN

Die Erfindung basiert auf der Erkenntnis, dass sich bestimmte technisch-physikalische Größen, die für die Reizung von Nerven- und Muskelgewebe erforderlich sind bei der induktiven Stimulation deutlich reduzieren lassen, wenn der zeitliche Verlauf des im Körper induzierten elektrischen Feldes und den dadurch verursachten Strömen dem dynamischen Verhalten der Ionentransportvorgänge in der Nervenzellmembran angepasst wird. Diese Größen können beispielsweise die benötigte Feldenergie, die Spulenverlustenergie, die erforderliche Spulenstromstärke, die erforderliche Spulenspannung, die maximale Steilheit der Spulenspannung oder des Spulenstromes oder der akustische Artefakt der Spule sein.

Weiterhin basiert die Erfindung auf der Erkenntnis, dass die entsprechenden induktiven Reizgeräte unterschiedliche zeitliche Verlaufsformen von Strom- und Spannung bei der Pulsabgabe erzeugen können müssen, um dadurch die Bedingungen für verschiedene Optimierungskriterien erfüllen zu können.

Figur 8 zeigt beispielhaft einen sehr günstigen zeitlichen Verlauf des Stromes für die Erregung einer Nervenzelle, der es ermöglicht mit einer geringen Amplitude oder Reizenergie ein Aktionspotential auszulösen. Insbesondere kann die in Figur 8 erkennbare erste dem eigentlichen positiven Stimulationspuls vorgelagerte negative Teil-Schwingung mit kleiner Amplitude durch ihre Anregung dynamischer Prozesse auf Membranebene die für eine Reizung erforderliche Amplitude des benötigten Stromes deutlich senken. Dies bedeutet beispielsweise, dass wenn die zugehörige Leistungselektronik einen zeitlichen Verlauf des Pulses so erzeugt, dass die Spulenspannung und damit auch das im Körper induzierte elektrische Feld einen Verlauf aufweisen, wie er in Figur 8 dargestellt ist, die benötigte Reizenergie reduziert werden kann.

Die Erkenntnis bezüglich der notwendigen zeitlichen Verlaufsformen des Feldes basiert auf einer mathematischen Modellierung der Nervenzellen, wie sie erstmalig von Hodgkin und Huxley aufgestellt wurden (A. L. Hodgkin, A. F. Huxley: A Quantitative Description of Membrane Current and its Application to Conduction and Excitation in Nerve. Journal of Physiology. 117, 1952, S. 500-544). Das Modell basiert auf einem Satz nichtlinearer Differentialgleichungen und simuliert das Verhalten von Nervenzellen, insbesondere das Verhalten kurzer Membranabschnitte von Axonen. Mit diesem Modell kann beispielsweise die Reaktion eines Axons auf von außen einwirkende elektrische Ströme nachvollzogen werden. Daher können hiermit die erforderlichen Reizströme bei verschiedenen Zeitverläufen der Pulse, die erforderlich sind, um ein Aktionspotential in der Nervenzelle auszulösen rechnerisch bestimmt werden.

Das Modell bezieht dabei das dynamische, nichtlineare Verhalten beispielsweise der Natriumund Kalium-Ionenkanäle der Zellmembran in die Simulation mit ein. Derartige Modelle beschreiben das zeitliche Verhalten von Neuronen mit nichtlinearen Termen hoher Ordnung und können insbesondere auch die Auslösung eines Aktionspotentials durch einen von außen aufgeprägten Strompuls unterschiedlicher Form simulieren. Eine direkte Invertierung der Gleichungen zur einfachen Bestimmung optimaler Pulsformen ist im Allgemeinen nicht möglich. Optimierungen der Pulsformen müssen somit über geschickte Abschätzungen mit anschließender quantitativer Bestätigung im Vorwärtsmodell erfolgen. Weiterhin erfordert eine genaue Simulation eines Auslösevorgangs der Depolarisation, während der kurzen Zeitspanne, in der das externe stimulierende Feld wirkt, Modifikationen und Erweiterungen der bisherigen Modelle. Insbesondere müssen hierfür die aktiven Nervenmodelle, um die elektrischen Eigenschaften des unmittelbar umgebenden Gewebes und um weitere Ionenkanal-Modifikationen, sowie um die elektrischen Eigenschaften der Spule erweitert werden. Allerdings kann durch Vergleich von Simulation und Experiment gezeigt werden, dass eine realistische Simulation dieses Axon-Verhaltens durchaus möglich ist.

Speziell in Bezug auf die Natrium-Ionenkanäle kann aus einem solchen Modell gefolgert werden, dass sowohl ein bahnender Mechanismus existiert, welcher die Auslösung eines Aktionspotentials begünstigt, als auch ein hemmender Mechanismus, welcher eine Auslösung eher unterdrückt. Diese beiden Mechanismen weisen ein stark unterschiedliches Zeitverhalten auf, was man sich für die Optimierung von energetisch besonders effektiven Reizen zunutze machen kann. Insbesondere kann aus der mathematischen Betrachtung dieser Mechanismen gefolgert werden, dass sich die Amplitude des für eine Reizung erforderlichen Strompulses beispielsweise senken lässt, wenn dem eigentlichen Stimulationspuls eine Teil-Schwingung mit kleiner Amplitude und entgegengesetzter Polarität vorgelagert wird, um den hemmenden Mechanismus abzuschwächen. Auf diese Weise können dynamische Prozesse auf Membranebene so angeregt werden, dass die für eine Reizung erforderliche Amplitude des Stimulationspulses und damit auch die erforderliche Pulsenergie, sowie die Stromwärmeverluste in der Spule deutlich gesenkt werden.

Weiterhin ist insbesondere eine Optimierung der Pulsform (z.B. auf minimale erforderliche Pulsamplitude oder -energie) infolge der großen Zahl veränderbarer Parameter extrem aufwändig. Außerdem kann bei einer solche Pulsformoptimierung noch hinsichtlich der die Pulsform definierenden Parameter noch unterschieden werden, ob die zu untersuchenden Stimulationspulse abschnittsweise sinusförmig sind oder nicht. Speziell abschnittsweise sinusförmige Stimulationspulse sind wesentlich einfacher zu simulieren und zu optimieren, da weniger Parameter verändert werden müssen. Weiterhin lassen sich solche abschnittsweise sinusförmige Signale auch technisch noch relativ einfach über eine Kombination resonanter Schwingkreise erzeugen. Dies ist in der unter dem amtlichen Aktenzeichen 10 2009 023 855.7 eingereichten Patentanmeldung erläutert.

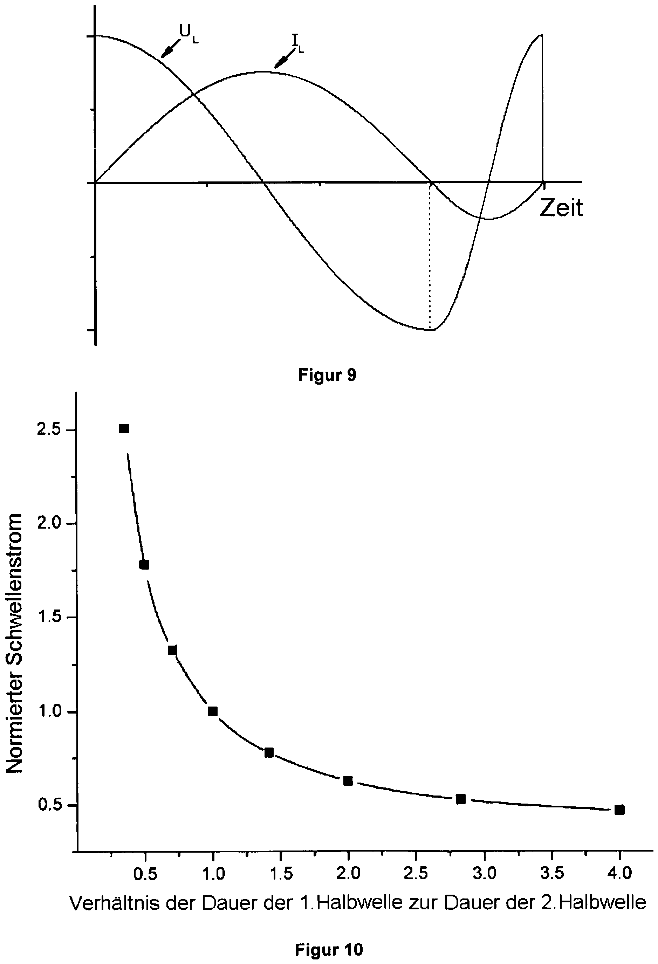

Figur 9 zeigt den Verlauf des im Gewebe induzierten elektrischen Stromes für einen Reizpuls, der sich aus zwei direkt aneinandergereihten Sinus-Halbwellen unterschiedlicher Frequenz zusammensetzt.

Figur 10 zeigt eine Effizienzbetrachtung solcher Pulse gemäß Figur 9, in Abhängigkeit des Frequenzverhältnisses von erster Halbwelle zu zweiter Halbwelle. Die Gesamt-Pulsdauer wurde hierbei konstant gehalten. Die Stromamplitude wurde dabei auf einen Quotienten von eins - entsprechend einer Sinusvollwelle als Pulsform - normiert. Man kann deutlich erkennen, dass der für eine Depolarisation der Nerven erforderliche Schwellenstrom sinkt, wenn die Dauer der ersten Halbwelle gegenüber der zweiten Halbwelle deutlich vergrößert wird, wie es beispielhaft in Figur 9 dargestellt ist. Daher kann bereits durch diese Modifikation der Pulsform eine wesentliche Energieoptimierung gegenüber bisherigen kommerziellen induktiven Stimulationssystem erreicht werden.

Allerdings ermöglicht eine weitergehende Betrachtung von Stimulationspulsen mit nichtperiodischem und nichtsinusförmigem Verlauf eine völlig freie Definition des Pulses mit nochmals deutlich gesteigertem Optimierungspotential. Hierbei kann gezeigt werden, das Pulse mit mehren Polaritätswechseln der Spulenspannung und damit des induzierten Feldes, sowie mit einem Einschwingverhalten, beginnend mit zunächst niedriger Amplitude nochmals eine deutliche Reduktion der erforderlichen Pulsenergie ermöglichen. Aus diesem Grund wurde ein Verfahren zur Ermittlung geeigneter optimierter Pulsformen, basierend auf einem Simulationssprozess entwickelt. Dabei wurde die gesamte Stimulationsumgebung einschließlich des Stimulators und eines einzelnen Nerven in ein Computer-Simulationsmodel aufgenommen.

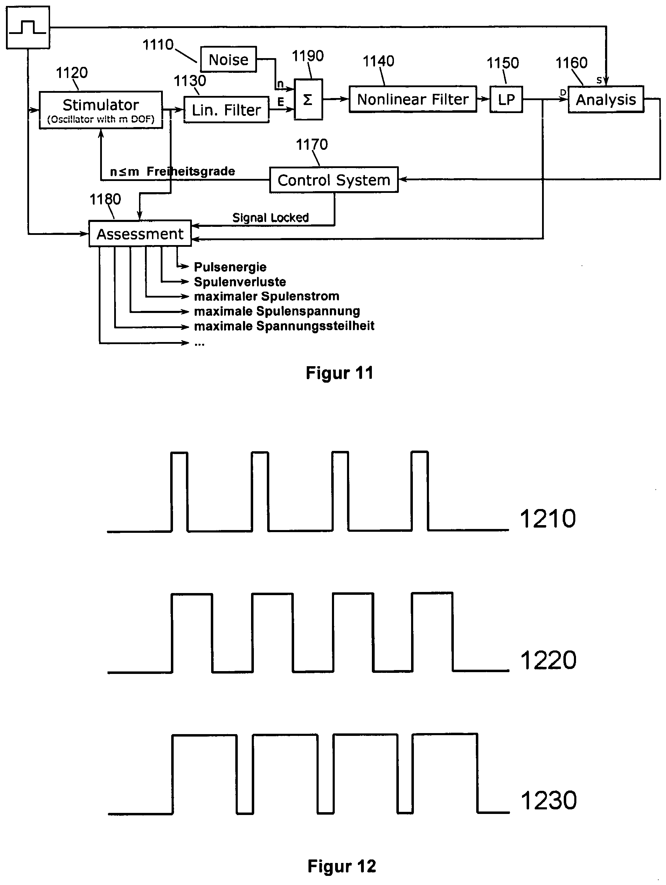

Figur 11 zeigt ein Blockschaltbild mit den entsprechenden Komponenten, wie sie zur Simulation einer Stimulationsumgebung zur Ermittlung optimaler Pulsformen eingesetzt werden kann, wobei diese Pulsformen von bevorzugten Ausführungsformen erzeugt werden. Basis einer solchen Nervensimulation kann beispielsweise ein Säugetier-Axon sein. Das Steuerungs-System der Simulation 1170 kann zur Optimierung der Pulsform nach unterschiedlichen Kriterien eingesetzt werden, wie z.B. Minimierung eines Parameters oder das Konstant-Halten einer Reaktion für einen parametrisierten Durchlauf des Stimulationssystems. Die Simulation des Stimulators 1120 kann dabei beispielsweise auf ein herkömmliches Stimulationssystem kalibriert werden, so dass hiermit gute Abschätzungen beispielsweise des thermischen Verhaltens von Stimulationsspulen bei Verwendung unterschiedlicher Pulsformen gemacht werden können.

Im Gegensatz zu früheren Arbeiten auf diesem Gebiet wurde hierbei das realitätsnahe, nichtlineare Nervenmodell eines Säugetiers, den bisher zu Grunde gelegten linearen Näherungen des Nervenmembran-Verhaltens vorgezogen, welches auf der dynamischen Beschreibung der spannungs-gesteuerten Kanal-Proteine basiert. Dieses Modell ist daher wesentlich besser geeignet, um den aktiven Prozess der Reizerzeugung zu analysieren. Insbesondere kann nur unter Anwendung eines aktiven Nervenmodells, das Mitwirken der spannungs-gesteuerten Kanäle zur Auslösung von Aktionspotentialen berücksichtigt werden, so dass sich hiermit noch deutlich fundiertere Pulsformoptimierungen ergeben, als mit passiven Modellen.

Weiterhin wird in Figur 11 das vollständige System zur Nerven- und Muskelreizung, zur Komplexitätsverringerung gegebenenfalls auf m Freiheitsgrade reduziert, implementiert. Beteiligte lineare Effekte wie die Induktion in das Zielgewebe können über Numerikverfahren wie z.B. die Finite Element Methode oder zur Vereinfachung auch durch analytische Formulierungen beschrieben werden. Im Blockschaltbild sind diese im Block Linear Filter 1130 zusammengefasst. Diese gefilterten Reizsignale wirken im Gewebe, ggf. zusammen mit Rauschprozessen wie sie der Körper und auch dessen Umgebung aufweist, auf das Reizobjekt, das mit seiner vollständigen nichtlinearen Dynamik und den Einkopplungsmechanismen bezüglich des induzierten Reizes im Block Nonlinear Filter 1140 abgebildet ist. Dabei kann sehr spezifisch eine möglichst genaue Abbildung des gewünschten Reizobjektes herangezogen werden. Soll beispielsweise ein alpha-Neuron im Nervus-Medianus-Bündel stimuliert werden, lässt sich hier die nichtlineare Beschreibung eines entsprechenden Axons einsetzen. Die Reaktion dessen lässt sich anschließend sowohl für die Regelung im Control System 1170 als auch zur Analyse der Stimulatoreinstellungen und -eigenschaften heranziehen.

Im folgenden soll beschrieben werden, mit welchen mathematischen Verfahren ein optimaler Zeitverlauf des Reizpulses entsprechend den Optimierungsparametern bestimmt werden kann:

**Korrelationsmethoden**

Das Ziel dieser Verfahren beruht darauf, ohne genauere Kenntnisse über ein nichtlineares dynamisches System dessen Verhalten mithilfe von Testmessungen durch eine Approximation mit für den entsprechenden Zweck hinreichender Genauigkeit abbilden zu können.

Wiener-*N* olterra-Reihendarstellung

Basierend auf den Arbeiten von N. Wiener und V. Volterra lässt sich beispielsweise eine Reihendarstellung für ein System darstellen, die die folgende oder eine äquivalente Form annimmt:

$$ s(t) = a_0 + \sum_{i=1}^{\infty} \frac{1}{i!} \cdot \int \ldots \int a_i(\tau_1, \tau_2, \tau_3, \ldots, \tau_i) \cdot x(t - \tau_1) x(t - \tau_2) x(t - \tau_3) \cdots x(t - \tau_i)\, d\tau_1 d\tau_2 d\tau_3 \cdots d\tau_i $$

Dies lässt sich sowohl auf einzelne Teile als auch auf das Gesamtsystem von Stimulator und Reizobjekt anwenden. Die Größe s(t) beschreibt in diesem Fall die neuronale Reaktion, x(t) den zeitlichen Verlauf des Stimulationsreizes; für eine Gesamtsystembeschreibung beispielsweise den Spannungsverlauf an der Stimulationsspule. Die Parameter $a_i$ (Kern) sind entsprechend zu bestimmen, dass sie das System möglichst genau wiedergeben. Je nach System und dessen Konvergenz bei dieser Reihendarstellung, kann die Entwicklung bereits nach einer sehr begrenzten Anzahl von Summanden abgebrochen werden, um höhere Glieder zu vernachlässigen. Beschränkt man die Darstellung beispielsweise lediglich auf die nullte und erste Ordnung mit $a_0$ und $a_1$, entspricht dies einer Linearisierung des Systems um einen entsprechenden Arbeitspunkt herum in typischer Sprungantwortbeschreibung. Die Parameter lassen sich auf vielerlei Arten aus Testmessungen so ermitteln, dass dieses Ensemble aus Teststimuli bestmöglich nachgebildet werden kann.

Als Eingangssignale zu Test- oder Systemanalysezwecken können dabei sehr gut variierende, stochastische oder pseudo-stochastische Prozesse eingesetzt werden, die schließlich jeweils ein bestimmtes Ausgangssignal im Modell hervorrufen. Dabei kann es sich beispielsweise um einen Rauschprozess handeln. Werden die Parameter des Eingangssignales (beispielsweise die Leistungsdichte als Maß für die Amplitude) als Steuergröße zur Regelung von Eigenschaften des Antwortsignales (beispielsweise eine bestimmte durchschnittliche Spitzenamplitude) herangezogen, lässt sich zusätzlich der Arbeitspunkt der Reihenentwicklung kontrollieren.

Als Ergebnis erhält man eine Abbildung des Systems, die in der auf wenige Summanden begrenzten Form deutlich weniger Komplexität besitzt als die ursprüngliche Modellabbildung. Die Parametersätze $a_i$ charakterisieren einerseits das Gesamtsystem als auch das Stimulationsobjekt deutlich genauer und differenzierter als alle bislang in der Elektrophysiologie gebräuchlichen Diagnoseverfahren. Zudem lassen sich auf diese Weise auch Vereinfachungen finden, die eine Invertierung und somit das Abschätzen des optimalen Eingangssignals für eine bestimmte Ausgangsreaktion erlauben. Diese Eigenschaft wiederum kann als Programmgrundlage für das neuartige Stimulationssystem herangezogen werden.

**Spike-Triggered Average, Spike-Triggered Covariance and Principal Component Analysis**

Eine weitere Methode zur Charakterisierung von Teilen oder des Gesamtsystems, bestehend aus Gerät und Reizziel, besteht in der Ermittlung des so genannten Spike-Triggered Averages (STA). Dies kann beispielsweise durch die Stimulierung mit Rauschen (optimalerweise weiß und Gauß-verteilt) erfolgen. Wenn ein solches Reizsignal einen Reaktionspuls auslöst, wird letzterer als Synchronisationsmarke verwendet. Wiederholt man diesen Vorgang über eine bestimmte Anzahl von Versuchen, lassen sich all diese Stimuli mit ihren jeweiligen Synchronisationsmarken durch Scharmittelung bzw. -summation zum STA formen, das somit einen Zeitverlauf für ein Reizsignal liefert, das Charakteristika aufweist, die alle erfolgreichen Stimuli in sich trugen.

$$\varphi_{STA}(t) = \sum_i s_i(t - \tau_i)$$

Dabei sind $s_i(t)$ die Stimuli und $\tau_i$ die von der Reaktion definierten Synchronisationsmarken. Auch hier lässt sich der Arbeitspunkt dieser Linearisierung über eine Regelung des Stimulationsprozesses beeinflussen. Bei weißem Gauß-Rauschen erfolgt dies am einfachsten über eine Nachregelung der Verteilungsbreite $\sigma$, um beispielsweise eine bestimmte Rate an Reaktionssignalen zu erhalten bzw. Reaktionen mit einer bestimmten Amplitude (beispielsweise halbes Sättigungsmaximum oder dergleichen) zu erzeugen.

Um weitere Informationen über das System zu erhalten, können die zueinander synchronisierten Stimuli auch einer Karhunen-Loeve-Transformation bzw. einer Principal Component Analysis im quantisierten Fall unterzogen werden. Als Ergebnis erhält man einen Satz aus Eigenvektoren und zugehörigen Eigenwerten, die das System am Arbeitspunkt charakterisieren. Liegt der Arbeitspunkt nahe der Auslöseschwelle der entsprechenden Nerven- oder Muskelzelle, gibt beispielsweise der Eigenvektor mit dem absolut größten Eigenwert jene Komponente in einer Wellenform an, die an dieser für die gleiche leichte Verstärkung den größten Einfluss auf die Reaktion in sich birgt. Entsprechend folgen die Eigenvektoren zu den nächstgrößeren Eigenwerten. Diese können auch in Linearkombinationen zusammen verwendet werden.

Als Varianzmaß zur STA eignet sich zur Diagnose die Spike-Triggered Covariance (STC). Sie stellt die Kovarianzmatrix der Einzelstimuli dar, nachdem von diesen die STA subtrahiert worden ist:

$$P_{s(t)}(response) = \frac{P(reponse)}{P\big(s(t)\big)} P_{response}\big(s(t)\big)$$

Optimierung mit Nebenbedingung

Mögliche Wellenformen können zudem aus Optimierungsalgorithmen gewonnen werden, die auf Teile des Systems oder den gesamten Aufbau angewandt werden. Gegenstand der Minimierung können dabei technische Größen sein, wie die für einen Puls benötigte Feldenergie, die Spulenverlustenergie, das Maximum des Betrages der erforderlichen Spulenstromstärke, das Maximum des Betrages der erforderlichen Spulenspannung, das Maximum des Betrages der zeitlichen Steilheit der Spulenspannung oder des Spulenstromes. Weiterhin kann sich die Optimierung aber auch auf eine durch induzierte Ströme verursachte minimale Gewebeerwärmung, eine maximale Reizwirkung bei einem vorgegebenen Maximalwert der Spulenspannung oder einen kleinstmöglichen akustischen Artefakt der Spule, also die empfundene Lautheit der Spule während des Pulses beziehen. Bei diesen genannten Optimierungen sollen Pulsformen ermittelt werden, die ein jeweils gleiches Reizergebnis, wie z.B. das Auslösen von Aktionspotentialen erzielen.

Zusätzlich kann auch der größtmögliche Sensitivitätsunterschied mehrerer räumlich nah beieinander liegender Zielstrukturen aus Zellgewebe für bestimmte Reize ermittelt werden, um eine optimale selektive Stimulation jeweils nur einer dieser Zelltypen vornehmen zu können. Insbesondere für die Reizung und Untersuchung von Neuronen im Gehirn ist zudem der Einsatz eines Optimierungsalgorithmus zum Ermitteln der nötigen Stimulationswellenform interessant, um spezielle Reaktionen mit besonderen Eigenschaften zu erhalten. Somit lassen sich beispielsweise spezielle Parameter für die optimierte Reizung bestimmter erkrankter Gewebestrukturen ermitteln, so dass Erkrankungen solcher Gewebestrukturen erkannt werden können.

Unter der Nebenbedingung, dass eine Zellreaktion (beispielsweise bestimmter Stärke) für das spezielle Stimulationsobjekt hervorgerufen wird, kann ein numerischer Optimierungsalgorithmus für ein entsprechendes Modell des Stimulationsobjektes und des Stimulators die entsprechend für die gewünschten Bedingungen optimale Wellenform ermitteln. Beispiel hierfür können bestimmte Eigenschaften des I-Wave-Patterns bestimmter Motoneurone des motorischen Cortex sein, die entsprechend in einer zu minimierende Zielfunktion formuliert und dem Algorithmus mit dem Modell übergeben werden können. Hierfür können einerseits lokale, meist gradientenbasiert arbeitende Algorithmen ebenso eingesetzt werden wie global oder pseudo-global suchende Verfahren. Letztere basieren oft auf Prinzipien von Mutation, Vererbung und Kombination oder Schwarmintelligenz.

Eine elektronische Schaltung gemäß der Erfindung, welche über eine Spule Pulse mit den oben beschriebenen Eigenschaften erzeugt, kann damit die jeweiligen oben beschriebenen Optimierungsziele erfüllen. Beispielsweise können Pulse erzeugt werden, die eine geringere magnetische Feldstärke und damit auch eine geringere Feldenergie zur Reizauslösung im Vergleich zu bisherigen Systemen für die induktive Magnetstimulation benötigen. Entsprechend können damit auch beispielsweise der erforderliche Spulenstrom und somit auch die Verluste der Spule, sowie ihre Erwärmung reduziert werden. Durch die Verringerung der Feldenergie kann weiterhin auch die zur Reizung benötigte Spulenspannung reduziert werden. Insbesondere ist es vorteilhaft, wenn die entsprechende elektronische Schaltung im Gegensatz zu bisherigen Systemen in der Lage ist, wahlweise unterschiedliche Pulsformen für die Spule zu erzeugen.

Für die technische Realisierung solcher Pulsformen kann allerdings nicht mehr auf ein resonantes Schaltungsprinzip, wie z.B. in Figur 6 dargestellt, zurückgegriffen werden. Auch ein Koppeln mehrere Schwingkreise, um ein aus mehreren Sinus-Teilschwingungen zusammengesetztes Pulssignal zu erzeugen, wäre wegen der hohen erforderlichen Pulsleistung für die Erzeugung solcher Pulsformen technisch zu aufwändig.

Im Prinzip könnten die erforderlichen Ströme und Spannungen für die Spule, wie sie zur Nervenreizung mit solchen Pulsformen benötigt werden nach dem Längsreglerprinzip erzeugt werden. Entsprechend einer bevorzugten Ausführungsform der Erfindung können daher über eine ausreichend hohe Versorgungsspannung und einen steuerbaren elektrischen Widerstand, der mit der Spule verbunden ist, beliebige, frei wählbare Verläufe des Spulenstromes erzeugt werden. Als steuerbarer Widerstand können dabei Leistungstransistoren eingesetzt werden. Durch Verwendung eines Massepotentials das beispielsweise mittig zwischen dem positivem und dem negativen Anschluss dieser Versorgungsspannung liegt, können sowohl positive als auch negative Spannungen in der Spule erzeugt werden. Allerdings erfordert dieses Längsreglerprinzip leistungsstarke, kostspielige Transistoren als steuerbare Widerstände. Weiterhin ist hier eine Rückspeisung der Feldenergie aus der Spule nur sehr bedingt möglich.

Um für nicht-sinusförmige Pulsformen, wie sie sich aus der oben beschriebenen Optimierung ergeben, die Möglichkeit der Rückgewinnung der magnetischen Feldenergie in einen Kondensator nutzen zu können, ist es vorteilhaft, hierfür die Stromrichtertechnik einzusetzen. Stromrichter sind Halbleiter-basierte Schaltungen der Leistungselektronik, die nach dem PWM-Prinzip arbeiten. D.h. diese Schaltungen basieren auf einem sehr schnellen An- und Abschalten einer entsprechenden Spannungsquelle, wobei der Stromverlauf zusätzlich durch die Induktivität einer Spule geglättet werden kann. Durch entsprechende Zeitmuster und die Verwendung mehrphasiger Systeme, sowie durch sogenannten Mehrquadrantenbetrieb kann auf diese Weise eine Umwandlung von Gleich- und Wechselstrom in nahezu beliebige Werte aus Strom und Spannung ermöglicht werden. Insbesondere kann ein solcher Stromrichter die elektrische Leistung zwischen zwei Verbrauchern/Erzeugern beliebig zwischen diesen beiden Komponenten hin- und herbefördern.

Somit wird erfindungsgemäß ein Stromrichter als Pulsquelle für die magnetische Neurostimulation eingesetzt . Dabei kann der Stromrichter in der Stimulationsspule einen nahezu frei wählbaren zeitlichen Stromverlauf erzeugen. Die Stimulationspule glättet dabei durch ihre Induktivität den vom Stromrichter geschalteten Stromverlauf. Gleichzeitig kann durch dieses Schaltungsprinzip die elektrische Energie von einem oder mehreren Speicherkondensatoren zur Spule und wieder zurückbefördert werden, so dass die magnetische Feldenergie der Spule zum größten Teil wieder zurückgewonnen werden kann.

Figur 12 zeigt drei beispielhafte Ausgangssignale eines PWM-Stromrichters 1210, 1220 und 1230 mit gleicher Taktfrequenz aber unterschiedlichem Tastverhältnis. Der Mittelwert des Ausgangssignals nimmt dabei vom Signal 1210 über 1220 nach 1230 zu. Bei entsprechend geringen Verlusten der verwendeten Halbleiterschalter kann somit der Mittelwert der Ausgangsspannung mit sehr geringen Verlusten beliebig variiert werden.

Konventionelle Stromrichter sind zwar in der Energietechnik sehr weit entwickelt, allerdings als Pulsquelle für optimierte Spulenpulse in der Magnetstimulation wenig geeignet. Dies liegt unter anderem daran, dass solche Systeme grundsätzlich für Dauerbetrieb (und damit z.B. zur Erzeugung periodischer Wechselstromsignale) und nicht für die Erzeugung kurzzeitiger Pulse ausgelegt sind. Daher wäre für ein Stimulationssystem ein entsprechend leistungsstarker Stromrichter mit einer Leistung von mehreren Megawatt erforderlich. Ein solcher Stromrichter wäre allerdings sehr groß und teuer, um als Medizingerät eingesetzt werden zu können. Ein weiterer Nachteil konventioneller Stromrichter liegt in der zeitlichen Auflösung: Um einen entsprechenden Stromverlauf des Spulenpulses durch PWM-Betrieb nachzubilden, muss der Stromrichter mit einer entsprechend hohen Frequenz schalten; allerdings haben Halbleiter mit der erforderlichen hohen Stromtragfähigkeiten meist relativ große Schaltzeiten, so dass ein solcher Stromrichter nicht schnell genug angesteuert werden könnte, um den erforderlichen zeitlichen Verlauf des Pulses ausreichend genau nachzubilden.

Weiterhin wurde bisher aber auch kein Anlass für den Einsatz von Stromrichtern für die magnetische Neurostimulation gesehen, da für die Pulsformoptimierung bisher nur extrem einfache lineare Nervenmodelle zu Grunde gelegt wurden, welche wiederum keinen Vorteil bei nichtsinusförmigen Wellenformen in Aussicht stellten.

Als Stromrichter für die optimierte induktive Magnetstimulation erweist sich insbesondere ein modifiziertes Konzept des modularen Multilevel Konverters (entsprechend der Offenlegungsschrift DE 101 03 031 von R. Marquardt) als besonders vorteilhaft. Dieser Stromrichter wird aus mehreren unabhängig voneinander steuerbaren Einzelmodulen aufgebaut, welche jeweils aus Leistungshalbleitern und Speicher-Kondensatoren aufgebaut sind. Jedes Einzelmodul besitzt zwei Leistungsanschlüsse und kann jeweils in beiden Polarisationsrichtungen im PWM-Betrieb geladen und entladen werden.

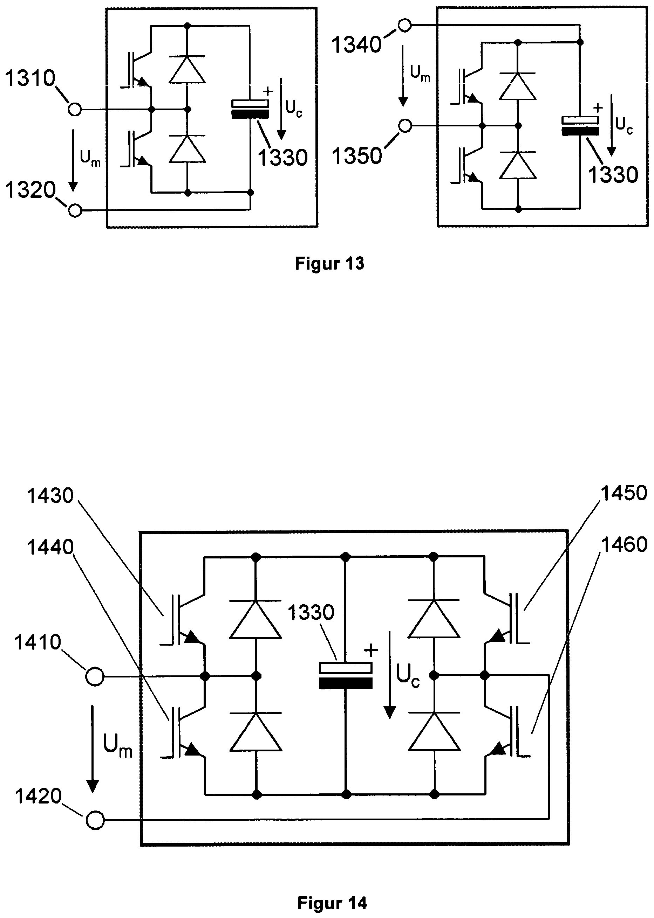

Figur 13 zeigt zwei Ausführungsformen von Halbbrückenmodulen eines Modularen Multilevel Konverters, wie sie in der Erfindung eingesetzt werden können. Durch entsprechendes Ansteuern der Halbleiterschalter kann das Modul an seinen Klemmen 1310 und 1320 bzw. 1330 und 1340 drei mögliche Zustände annehmen: Einen Schaltzustand in dem die Klemmenspannung des Moduls unabhängig von der Klemmenstromrichtung den Wert Null annimmt (bis auf den Durchlass-Spannungsabfall realer Halbleiter); einen Schaltzustand in dem die Klemmenspannung des Moduls unabhängig von der Klemmenstromrichtung von Null verschiedene Werte annimmt und das Subsystem - je nach Klemmenstromrichtung - regenerativ Energie aus dem Speicherkondensator 1330 aufnehmen oder abgeben kann; und einen Schaltzustand in dem die Klemmenspannung des Moduls abhängig von der Klemmenstromrichtung so gerichtet ist, dass das Modul keine Energie abgibt.

Figur 14 zeigt ein einzelnes Vollbrückenmodul eines Modularen Multilevel Konverters, wie es in der Erfindung eingesetzt werden kann. Im Gegensatz zu den Halbbrückenmodulen kann dieses Modul auch die Klemmenspannung invertieren, wobei auch dann nicht die Polarität des Speicherkondensators 1330 invertiert werden muss. Daher kann durch entsprechendes Ansteuern der Halbleiterschalter 1430, 1440, 1450 und 1460 dieses Modul sowohl beim Laden als auch beim Entladen des Kondensators 1330 Spannungen und Ströme beider Polaritäten bearbeiten (4-Quadrantenbetrieb).

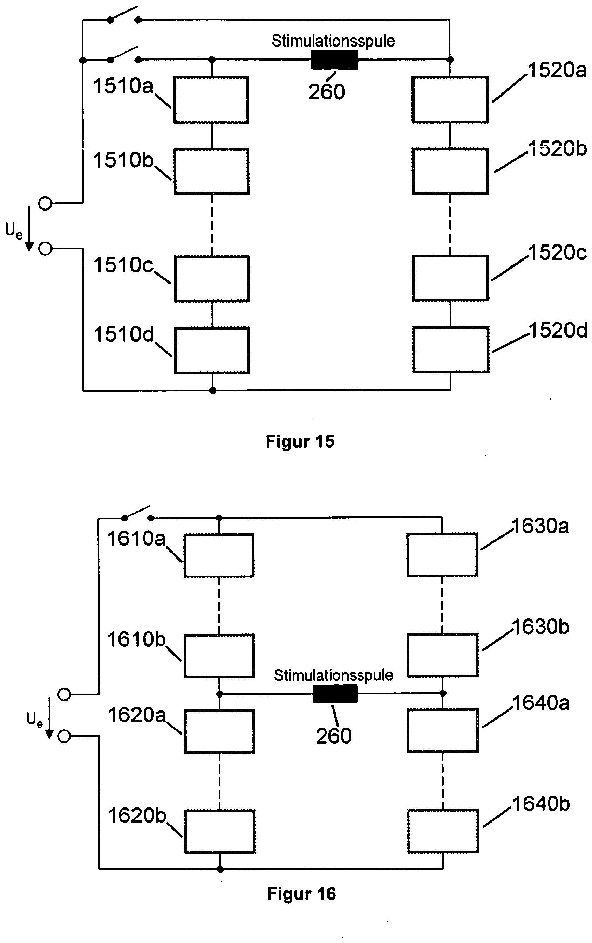

Figuren 15 und 16 zeigen bevorzugte Ausführungen der Erfindung am Beispiel einer Verschaltung mit zweisträngiger bipolarer Speisung der Stimulationsspule 260. Sämtliche Module 1510a bis 1520d bzw. 1610a bis 1640b können im Multiplex-Betrieb mit einer niedrigen Versorgungsspannung geladen werden. Da bei Verwendung von Vollbrückenmodulen gemäß Figur 14 jedes Modul durch entsprechendes Ansteuern der Modul-Transistoren seine Spannungspolarität invertieren kann, muss jeder Strang nur die halbe Spitzenspannung aufbringen können.

Die Verwendung dieses Stromrichtersystems auf Basis des Modularen Multilevel Converters weist folgende für die Realisierung einer Pulsquelle eines induktiven Stimulators wichtige Vorteile auf:

Durch Verwendung entsprechend dimensionierter Kondensatoren in jedem Einzelmodul, kann der Stromrichter selbst die erforderlichen Kurzzeit-Energiespeicher zur Entlastung der Netzstromversorgung in Form von Kondensatoren 1330 bereitstellen. Diese Kondensatoren können daher den großen Pulskondensator 220 bisheriger Geräte zur induktiven Neurostimulation ersetzen.

Durch Verwendung vieler seriell geschalteter Module tritt in jedem einzelnen Modul nur eine relativ geringe Spannung auf, so dass preisgünstige Kondensatoren und Halbleiterbauelemente eingesetzt werden können. Insbesondere können in diesem Fall besonders effiziente MOSFET-Transistoren mit relativ geringer Spannungsfestigkeit, sehr kurzen Schaltzeiten und sehr geringen Durchlassverlusten eingesetzt werden.

Der Einsatz von MOSFET-Transistoren als schaltende Bauelemente wiederum ermöglicht ein Auslegen des Stromrichters für kurzzeitige Strom- und Leistungsspitzen (während der Pulsphase), da diese Bauteile kurzzeitig mit deutlich höheren Strömen beaufschlagt werden können, als im Dauerbetrieb. Damit kann ein solcher Stromrichter mit kleineren, kostengünstigeren Halbleiterbauelementen aufgebaut werden als ein Stromrichter, der für Dauerbetrieb ausgelegt sein soll. Ein weiterer Vorteil des Einsatzes von MOSFET-Transistoren liegt darin, dass sich diese Bauelemente relativ einfach parallel schalten lassen. Dadurch kann über die Parallelschaltung vieler relativ kostengünstiger MOSFET-Transistoren aus der Consumer-Elektronik erreicht werden, dass die für die Spule erforderlichen hohen Ströme geschaltet werden können.

Da innerhalb der Module nur eine Spannungsrichtung auftritt, können in diesen Modulen - im Gegensatz zu herkömmlichen resonanten Pulsquellen für die Magnetstimulation - auch kleine kostengünstige Elektrolytkondensatoren mit sehr kleinem Bauvolumen eingesetzt werden, wodurch die Baugröße der Pulsquelle verringert werden kann.

Die Ausgangsspannung und damit die Spulenspannung kann beim modularen Multilevel Konverter nicht nur über das Tastverhältnis des PWM-Signals, sondern auch über die Spannung und Anzahl der momentan aktiven Einzelmodule gesteuert werden, da sich die Spannungen der einzelnen aktiv geschalteten Module zu einer Gesamtspannung an der Spule addieren oder subtrahieren lassen. Hierdurch wiederum kann ein deutlich glatterer Verlauf der Spulenspannung erreicht werden als bei alleiniger Verwendung des PWM-Prinzips konventioneller Stromrichter.

Durch asynchrones Schalten der vielen Einzelmodule des modularen Multilevel Konverters kann die Erzeugung eines Ausgangssignals mit deutlich feinerer zeitlicher Stufung als bei einem reinen PWM-Signal erreicht werden. Somit kann bezüglich des Spulenstromes eine wesentlich feinere zeitliche Auflösung des erzeugten Ausgangssignals an der Spule als bei einem konventionellen Stromrichter erzielt werden.

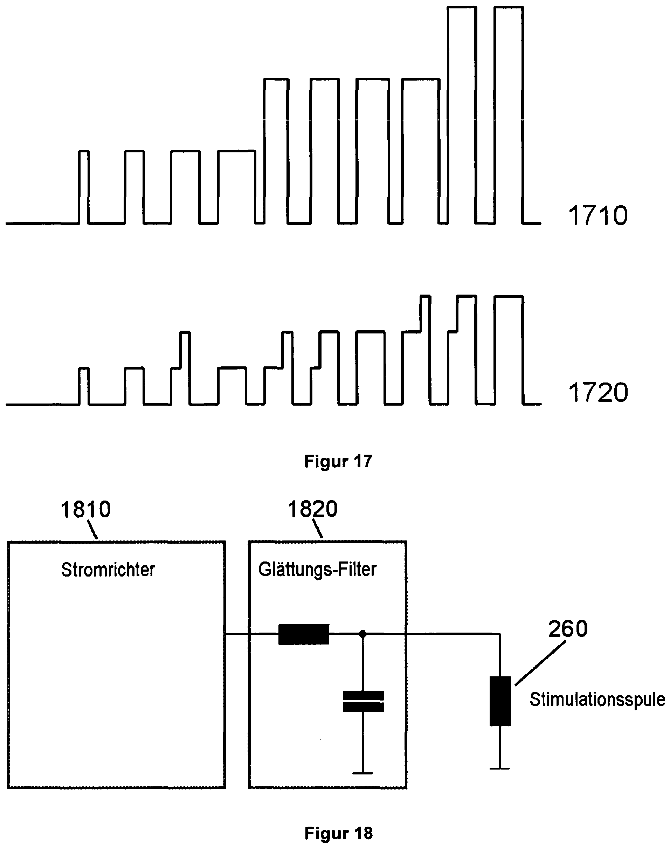

Figur 17 zeigt beispielhaft PWM-Ausgangssignal-Verläufe, wie sie durch einen Modularen Multilevel Konverter unter Verwendung mehrerer Einzelmodule mit insgesamt 3 Spannungsniveaus bei verschiedenen Tastverhältnissen erzeugt werden können. Beim Signal 1710 werden nacheinander erst ein Modul, dann zwei und schließlich 3 Module synchron geschaltet. Beim Signal 1720 werden beispielhaft drei Module asynchron geschaltet, um feinere Stufungen zu erreichen.

Weiterhin kann in einer bevorzugten Ausführungsform der Erfindung zwischen die Leistungselektronik zur Erzeugung des Spulenpulses und die Spule noch eine geeignete Glättungsschaltung eingefügt werden, die den Verlauf der Spulenspannung und des Spulenstromes gegenüber einem geschalteten Signal, z.B. einem PWM-Signal glättet.

Fig. 18 zeigt beispielhaft eine Glättungsschaltung, die beispielsweise als Tiefpassfilter direkt zwischen den Ausgang eines Stromrichters 1810 und die Stimulationsspule 260 eingefügt werden kann, um den Verlauf der Spulenspannung und des Spulenstromes gegenüber dem PWM-Signal zu glätten.

Weiterhin werden in einer bevorzugten Ausführungsform der Erfindung die einzelnen Module des Modularen Multilevel Konverters so angesteuert, dass auch mit einer niedrigen externen Versorgungsspannung eine hohe Pulsspannung an die Stimulationsspule abgegeben werden kann: Im Lade-Modus können die Module eines Stranges durch entsprechendes Ansteuern ihrer jeweiligen Leistungshalbleiter so geschaltet werden, dass beispielsweise jeweils nur der Kondensator eines Moduls mit der externen Spannungsversorgung verbunden ist und die übrigen Module nach außen eine direkte Kurzschluss-Verbindung darstellen. Auf diese Weise können sequentiell die Speicherkondensatoren der Einzelmodule aufgeladen werden. Hierdurch wiederum kann das Gesamtsystem mit relativ geringer Spannung versorgt und betrieben werden. Für die Abgabe der Pulse bei großen Spannungen, können die Module seriell geschaltet eine Spannung entsprechend der Summe ihrer einzelnen Kondensatorspannungen erzeugen. Das bei bisherigen Geräten zur induktiven Neurostimulation benötigte aufwändige Hochspannungsnetzteil kann somit entfallen, wodurch die Baugröße der Pulsquelle verringert werden kann.

Damit ermöglicht die Verwendung eines Stromrichters basierend auf dem Konzept des modularen Multilevel Konverters gemäß bevorzugter Ausführungsformen die Einsparung vieler Komponenten bisheriger resonanter Leistungskreise.

Insbesondere ermöglicht der Einsatz eines Stromrichters, wie er beschrieben wurde, dass über ein einziges Gerät magnetische Pulse über eine Spule zur Reizung von Zellen mit nahezu beliebiger zeitlicher Verlaufsform erzeugt werden können.

Somit sind bevorzugte Ausführungsformen der Erfindung so ausgelegt, dass - vom Benutzer wählbar - Pulse unterschiedlicher Form generiert werden können, welche die oben beschriebenen jeweiligen Optimierungskriterien erfüllen.

Beispielsweise werden optimierte Pulse so erzeugt, dass sie bevorzugt ganz bestimmte Typen von Nerven- oder Muskelzellen optimal reizen. Durch Anpassung der Form der Pulse speziell auf bestimmte Typen von Nerven- und/oder Muskelzellen, können diese Nervenzellen mit einer geringstmöglichen Reizschwelle bevorzugt gegenüber anderen Typen von Nerven- und/oder Muskelzellen erregt werden.

Bei einer weiteren bevorzugten Ausführungsform können Pulse mit beliebig wählbarem zeitlichen Verlauf an die Spule abgegeben werden. Der zeitliche Verlauf des Spulenstromes oder der Spulenspannung kann dabei entweder direkt als Analogsignal oder in Form digitaler Daten von der Pulsquelle eingelesen werden.

Weiterhin ermöglicht der Einsatz eines Stromrichters gemäß der Erfindung, wie er beschreiben wurde, dass über dasselbe Gerät auch Pulse abgegeben werden können, welche eine geringe Geräuschentwicklung in der Spule zur Folge haben. Da die magnetischen Kräfte der Spulenleiter während des Pulses kurzzeitige Verformungen der Spule zur Folge haben, machen sich diese Verformungen als teilweise sehr lautes Klickgeräusch bemerkbar. Dieser sogenannte akustische Artefakt der Magnetstimulation kann neurologische Untersuchungen empfindlich stören. Beispielsweise zeigen Pulse mit einem geringen Anteil hoher Frequenzen einen deutlich geringeren akustischen Artefakt; allerdings erfordern diese Pulse mehr Energie bei gleicher Wirkung, so dass die Spule schneller erwärmt. Bisherige Geräte, die nur eine feste Pulsform abgeben können, konnten daher nicht auf möglichst geringen akustischen Artefakt hin optimiert werden.

Weiterhin ermöglicht der Einsatz eines Stromrichters gemäß der Erfindung, wie er beschreiben wurde, dass über das selbe Gerät alternativ Pulse abgegeben werden können, die bei gegebener Maximalspannung eine möglichst hohe Reizwirkung erzielen. Die beschriebenen Optimierungen hinsichtlich der Effizienz der Pulse hat zwar zur Folge, dass die Pulse bei gegebener Reizwirkung möglichst wenig Energie verbrauchen; allerdings bedeutet dies nicht, dass solche Pulse bei vorgegebener Maximalamplitude der Spulenspannung auch ein maximales Reizergebnis zur Folge haben.

Ein weiterer Vorteil, der sich aus dem Einsatz eines Stromrichters zur Erzeugung der Pulse für die Spule ergibt, liegt darin, dass - im Gegensatz zu bisher verwendeten induktive Reizgeräten - die Form des Pulses unabhängig von der Induktivität der eingesetzten Spule ist.

Durch entsprechend geschickte Ausführungsformen wie beispielsweise der Einsatz von gleichrichtenden Schaltelementen, etwa Dioden in den Einzelmodulen in den Figuren 13 und 14, lässt sich das bei unbekannten Stimulationsspulen auftretende Problem von vorab nicht bekannten elektrischen Eigenschaften und deren Einfluss auf die Pulsform ferner durch die Verwendung eines weiteren Schaltungszustandes vereinfacht lösen. Eine beispielsweise aus oben genanntem Grund am Ende eines Pulses noch im magnetischen Feld der Spule vorhandene Energie kann auf diese Weise ohne weitere Kenntnisse der Spuleninduktivität gezielt ohne Gefahr abgebaut werden, indem ein passiver Spannungsverlauf nahezu beliebiger Verlaufsform vorgegeben wird, dessen Polarität jedoch entweder - im Falle der Dioden - nicht vorgegeben wird oder beispielsweise durch eine Steuerung vom Spulenstrom bzw. dessen Zeitableitung bedingt wird. Dies legt den Leistungsfluss unter fest definierten sonstigen Freiheitsgraden fest, der somit beispielsweise zurück in die Speicherelemente der Module erzwungen werden kann, ohne unkontrollierte Entladungen oder eine Umsetzung in Wärme zu verursachen. Kenntnisse über den letzten Stromverlauf, die Induktivität oder Verlustwiderstände sind hierzu im einfachsten Fall nicht zwingend erforderlich. Für den möglichen Spannungsverlauf und dessen Darstellung beispielsweise über Pulsweitenmodulation gelten hierbei im Allgemeinen dieselben Randbedingungen wie für den aktiven, polaritätsdefinierenden Betrieb. In der Ausführungsform über gleichrichtende Schaltelemente erlischt der Stromfluss automatisch, sobald sämtliche elektromagnetische Restenergie aus der Stimulationsspule und anderen Induktivitäten entladen ist.

Insbesondere kann ein Stromrichter-basiertes Stimulationssystem auch für die Optimierung oder Analyse bestimmter Pulsformen direkt am Zielobjekt in vivo, beispielsweise an Gehirn- oder peripheren Neuronen eingesetzt werden. Es kann dabei entweder nur ein Teil wie beispielsweise das Neuron oder das gesamte System aus Stimulator, Spule und Neuron als zu analysierendes System betrachtet werden. Ein- und Ausgangssignal sind dementsprechend zu definieren.

Im Gegensatz zu bisherigen Geräten zur induktiven Nervenstimulation ist ein Stromrichter-basiertes Gerät in der Lage die hierfür nötigen Reize unterschiedlicher Pulsformen zu generieren und den oben beispielhaft beschriebenen Regelverfahren zum Auffinden optimaler Pulsformen im Betrieb dynamisch anzupassen. Ebenso ist das Stimulatorprinzip in der Lage, Rauschsignale zu erzeugen.

Um typische Messfehler und -varianzen auszugleichen, sollte gegebenenfalls das jeweilige Abbildungspaar aus Eingangs- und Ausgangssignal für den Regelalgorithmus physikalisch mehrmals gemessen und durch Mittelung im Signal-zu-Rausch-Verhältnis verbessert werden.

Somit erlaubt das erfindungsgemäße Stimulatorprinzip, welches in der Lage ist, beliebige Pulsformen zu erzeugen, auch verschiedene Untersuchungen von Nerven- und Muskelzellen in der Elektrophysiologie und der Psychiatrie. Im Gegensatz zu Untersuchungsmethoden wie der Analyse der reinen Reizschwelle oder der peripheren Nervenreizleitgeschwindigkeit (NCS, Nerve Conduction Study) und deren Veränderungen, bieten die oben beschriebenen Verfahren eine vollständige Charakterisierung eines Neurons an. Auf diese Weise lässt sich somit ein deutlich größeres Feld an neuronalen Läsionen diagnostisch sehr einfach - nach entsprechender Analyse über verschiedene Pulsformen - durch Veränderungen der entsprechenden Parameter charakterisieren und klassifizieren. Je nach Verfahren sind die Nervenreizleitgeschwindigkeit und die Reizschwelle dabei sogar aus den Parametern ohne weitere Messungen direkt errechenbar.

Ein weiteres Einsatzgebiet ist die differenzierte Optimierung der Stimulatorparameter (d.h. die zeitliche Verlaufsform des Pulses) direkt am Objekt. Dies kann direkt an einer Person stattfinden oder in Näherung für eine Vielzahl von unterschiedlichen Reizobjekten vorab geschehen, damit diese dann bei der eigentlichen Anwendung in einer Datenbank zur Verfügung stehen und entsprechend eingesetzt werden können. Dabei lassen sich die Pulsformen nicht nur auf bestimmte unterschiedliche Zelltypen wie Muskelzellen oder Neuronen oder auch spezielle Subtypen der entsprechenden Klasse anpassen, sondern auch auf Teile einzelner Zellen, die elektrisch unterschiedliche Eigenschaften besitzen. Bei Neuronen wären beispielsweise Dendriten, Soma, Axon und Synapsen zu nennen, die aufgrund ihres unterschiedlichen elektrischen Verhaltens entsprechend andere Pulsformen zur Reizung - beispielsweise bei optimal niedriger Schwelle - benötigen. Insbesondere lässt sich damit auch vorteilhaft ein System zur Ermittlung einer optimierten Pulsform, basierend auf den Rechenverfahren, wie sie oben beschrieben wurden in das beschriebene Stimulationsgerät zur Erzeugung beliebiger Wellenform integrieren.

Der Einsatz von magnetischen Pulsen nach dem beschriebenen Verfahren kann zur Reizung von Nerven- und Muskelzellen eingesetzt werden. Insbesondere kann es auch für den gezielten Muskelaufbau oder die Darstellung funktionaler Zusammenhänge des neuromotorischen Systems bei Menschen und Tieren eingesetzt werden. Die Dauer der von der Spule abgegebenen magnetischen Feldpulse liegen etwa im Bereich von 20 bis 3000 Mikrosekunden; vorzugsweise sollte die Dauer im Bereich von 100 bis 1000 Mikrosekunden liegen.

Die Stärke der magnetischen Feldpulse sollte an der Spulenoberfläche im Bereich einer Flußdichte von 0,1 bis 5 Tesla liegen. Vorzugsweise sollte die magnetische Flußdichte im Bereich von 0,3 bis 1 Tesla liegen.

**Patentansprüche**

1. Vorrichtung zur Erzeugung kurzer starker Strompulse in einer Spule (260), so dass die Spule magnetische Feldpulse erzeugt, die nach dem Prinzip der elektromagnetischen Induktion im Körpergewebe elektrische Reizströme hervorrufen, die ein Aktionspotential zur Reizung von Nerven- und/oder Muskelzellen auslösen,

wobei die Spule so ausgeführt ist, dass sie nahe bei dem zu reizenden Körpergewebe positionierbar ist, damit ein von ihr erzeugtes Magnetfeld das Körpergewebe durchsetzt; wobei die Vorrichtung mindestens einen Kondensator (1330) zur Speicherung und Abgabe der für die magnetischen Feldpulse benötigten Energie und eine geeignete Ladeschaltung zum Aufladen dieses mindestens eines Kondensators enthält; und

wobei die Vorrichtung eine Stromerzeugungseinheit umfasst, die eingerichtet ist, einen frei wählbaren zeitlichen Verlauf des Stromes durch die Spule während des Strompulses zu erzeugen und wobei die Vorrichtung eingerichtet ist, einen optimierten zeitlichen Verlauf des Stromes durch die Spule so zu steuern, dass das von der Spule induzierte elektrische Feld im Gewebe einen Strom zur Folge hat, dessen Zeitverlauf an die dynamischen Ladungstransportphänomene der Nervenfasern angepasst ist, um eine benötigte Feldstärke oder Feldenergie zur induktiven Reizung der Nerven- und/oder Muskelzellen zu reduzieren oder um jeweils nur einen Zelltyp von mehreren räumlich nah beieinander liegenden Zielstrukturen aus Zellgewebe optimal selektiv zu stimulieren , **dadurch gekennzeichnet, dass** zur Erzeugung des zeitlichen Verlaufes des Spulenstromes ein modularer Multilevel Konverter, welcher aus mehreren unabhängig voneinander steuerbaren Einzelmodulen aufgebaut ist, eingesetzt wird.

2. Vorrichtung nach Anspruch 1, wobei mindestens zwei Kondensatoren (1330) zur Speicherung und Abgabe der für die Feldpulse benötigten Energie eingesetzt werden und die Kondensatoren über geeignete Leistungshalbleiter wahlweise so mit der Spule (260) elektrisch verbunden werden, dass unterschiedliche Spannungen an der Spule entstehen und so ein bestimmter zeitlicher Verlauf des Stromes durch die Spule erzielt wird und wobei zur Erzeugung des zeitlichen Verlaufes des Stromes durch die Spule über das elektrische Verbinden von Kondensatoren mit der Spule über geeignete Leistungshalbleiter ein modularer Multilevel Konverter bestehend aus mindestens zwei in Reihe geschalteten Halb- oder Vollbrückenmodulen (1510, 1520; 1610, 1620) eingesetzt wird.

3. Vorrichtung nach Anspruch 1 oder 2, wobei zur Erzeugung des zeitlichen Verlaufs des Stromes durch die Spule (260) Leistungshalbleiter eingesetzt werden, die pulsweitenmodulierte Signale erzeugen und wobei ein Stromrichter zur Erzeugung der pulsweitenmodulierten Signale eingesetzt wird.

4. Vorrichtung nach Anspruch 3, wobei als Stromrichter ein modularer Multilevel Konverter bestehend aus mindestens zwei in Reihe geschalteten Halb- oder Vollbrückenmodulen (1510, 1520; 1610, 1620) eingesetzt wird und wobei die Kondensatoren (1330) einzelner Halb- oder Vollbrückenmodule des modularen Multilevel Konverters als Energiespeicher für die Bereitstellung des Reizpulses eingesetzt werden.

5. Vorrichtung nach einem der Ansprüche 1-4, wobei der zeitliche Verlauf des Stromes durch die Spule (260) hinsichtlich mindestens eines Parameters optimiert wird, wobei der Parameter eine auf einen Strompuls bezogene durch Stromwärmeverluste verursachte minimale Verlustenergie in der Spule, eine auf einen Strompuls bezogene durch Stromwärmeverluste verursachte minimale Verlustenergie eines gesamten Leistungskreises, ein kleinstmögliches Maximum des Betrages der Spulenstromstärke, ein kleinstmögliches Maximum des Betrages der Spulenspannung, ein kleinstmögliches Maximum des Betrages der zeitlichen Steilheit des Spulenstromes, ein kleinstmögliches Maximum des Betrages der zeitlichen Steilheit der Spulenspannung, eine auf einen Strompuls bezogene minimale Wirbelstromverlust-Energie im Körpergewebe, eine maximale Reizwirkung bei einem vorgegebenen Maximalwert der Spulenspannung, eine maximale Reizwirkung bei vorgegebener in einem oder mehreren Speicherkondensatoren gespeicherter Energie oder die durch den akustischen Artefakt verursachte Lautheit des Spulengeräusches sein kann.

**Claims**

1. An apparatus for generating short strong current pulses in a coil (260) such that the coil produces magnetic field pulses which, according to the principle of electromagnetic induction, cause electrical currents for stimulation in body tissue which trigger an action potential for stimulating nerve and/or muscle cells,

wherein the coil is configured to be positioned close to the body tissue to be stimulated so that a generated magnetic field permeates the body tissue;
wherein the apparatus includes at least one capacitor (1330) for storing and outputting the energy required for the magnetic field pulses and a suitable charging circuit for charging said at least one capacitor; and
wherein the apparatus comprises a power generation unit arranged to generate a freely selectable time behaviour of the current through the coil during the current pulse and wherein the apparatus is arranged to control an optimized time behaviour of the current through the coil such that the electric field induced by the coil in the tissue results in a current the time behaviour of which is matched to the dynamic charge transport phenomena of the nerve fibres, in order to reduce a required field strength or field energy for inductive stimulation of the nerve and/or muscle cells or in order to optimize selective stimulation of only one cell type of a plurality of target structures of cell tissue closely adjacent to each other at a time,

**characterized in that**

for generating the time behaviour of the coil current, a modular multilevel converter is used, which consists of a plurality of individual modules that can be controlled independently of each other.

2. The apparatus according to claim 1, wherein at least two capacitors (1330) are used for storing and outputting the energy required for the field pulses and the capacitors are selectively electrically connected to the coil (260) via suitable power semiconductors so that different voltages are generated at the coil and a specific time behaviour of the current through the coil is achieved, and

   wherein for generating the time behaviour of the current through the coil by electrically connecting the capacitors to the coil via suitable power semiconductors a modular multilevel converter is used which consists of at least two half-bridge or full-bridge modules (1510, 1520; 1610, 1620) connected in series.

3. The apparatus according to claim 1 or 2, wherein four generating the time behaviour of the current through the coil (260) power semiconductors are used which produce pulsewidth modulated signals and wherein a current converter is used for producing the pulsewidth modulated signals.

4. The apparatus according to claim 3, wherein as the current converter a modular multilevel converter is used which consists of at least two half-bridge or full-bridge modules (1510, 1520; 1610, 1620) connected in series, and wherein the capacitors (1330) of individual half-bridge or full-bridge modules of the modular multilevel converter are used as energy storage means for providing the stimulation pulse.

5. The apparatus according to any one of claims 1 - 4, wherein the time behaviour of the current through the coil (260) is optimized with respect to at least one parameter, the parameter being a minimum energy loss in the coil caused by joul heat loss related to a current pulse, a minimum energy loss of an entire power circuit caused by joul heat loss related to a current pulse, a smallest possible maximum of the magnitude of the coil current intensity, a smallest possible maximum of the magnitude of the coil voltage, a smallest possible maximum of the magnitude of the slope of the coil current over time, a smallest possible maximum of the magnitude of the slope of the coil voltage over time, a minimum eddy current loss energy in body tissue related to a current pulse, a maximum stimulating effect at a predetermined maximum value of the coil voltage, a maximum stimulating effect at predetermined energy stored in one or more storage capacitors, or the loudness of the coil noise caused by the acoustic artifact.

**Revendications**

1. Dispositif de génération d'impulsions de courant courtes et fortes dans une bobine (260), de sorte que la bobine génère des impulsions de champ magnétique qui, en fonction du principe d'induction électromagnétique dans le tissu corporel, provoquent des courants de stimulation électrique, qui déclenchent un potentiel d'action pour stimuler les cellules nerveuses et/ou musculaires,

   la bobine étant conçue, de sorte qu'elle peut être positionnée près du tissu corporel à stimuler, de sorte qu'un champ magnétique généré par celle-ci imprègne le tissu corporel ; le dispositif renfermant au moins un condensateur (1330) pour stocker et décharger l'énergie requise pour les impulsions de champ magnétique et un circuit de charge approprié pour charger ce au moins un condensateur ; et
   le dispositif comprenant une unité de génération de courant, qui est conçue, pour générer une courbe de courant dans le temps pouvant être librement sélectionnée à travers la bobine durant l'impulsion de courant, et le dispositif étant conçu, pour commander une courbe de courant dans le temps optimisée à travers la bobine d'une manière telle que le champ électrique induit par la bobine a comme conséquence un courant dans le tissu, dont la courbe dans le temps est adaptée au phénomène de transport de charges dynamiques des fibres nerveuses, afin de réduire une intensité de champ ou une énergie de champ requise pour la stimulation inductive des cellules nerveuses et/ou musculaires ou respectivement afin de stimuler sélectivement de manière optimale uniquement un type de cellule d'une pluralité de structures cibles composées de tissu cellulaire situées à proximité spatiale les unes des autres, **caractérisé en ce que**, afin de générer la courbe de courant de la bobine dans le temps, un convertisseur multiniveau modulaire, qui est composé d'une pluralité de modules individuels qui peuvent être commandés indépendamment l'un de l'autre, est utilisé.

**2.** Dispositif selon la revendication 1, au moins deux condensateurs (1330) étant utilisés pour le stockage et la décharge de l'énergie requise pour les impulsions de champ, et les condensateurs étant connectés sélectivement électriquement à la bobine (260) par l'intermédiaire de semi-conducteurs de puissance, de sorte que différentes tensions sont produites au niveau de la bobine et ainsi qu'une courbe spécifique du courant dans le temps est atteinte à travers la bobine et pour générer la courbe de courant dans le temps à travers la bobine par l'intermédiaire de la connexion électrique des condensateurs à la bobine par l'intermédiaire de semi-conducteurs de puissance appropriés, un convertisseur multiniveau modulaire constitué d'au moins deux modules en demi-pont ou en pont complet (1510, 1520 ; 1610, 1620) connectés en série est utilisé.

**3.** Dispositif selon la revendication 1 ou 2, pour générer la courbe de courant dans le temps à travers la bobine (260), sont utilisés des semi-conducteurs de puissance qui génèrent des signaux modulés en largeur d'impulsion, et un convertisseur d'énergie est utilisé pour générer les signaux modulés en largeur d'impulsion.

**4.** Dispositif selon la revendication 3, un convertisseur multiniveau modulaire constitué d'au moins deux modules en demi-pont ou en pont complet (1510, 1520 ; 1610, 1620) connectés en série étant utilisé comme convertisseur de puissance et les condensateurs (1330) des modules individuels en demi-pont ou en pont complet du convertisseur multiniveau modulaire étant utilisés comme accumulateur d'énergie pour fournir l'impulsion de stimulation.

**5.** Dispositif selon l'une des revendications 1 à 4, la courbe de courant dans le temps à travers la bobine (260) étant optimisée par rapport à au moins un paramètre, le paramètre pouvant être une énergie de perte minimale dans la bobine liée à une impulsion de courant due aux pertes thermiques de courant, une énergie de perte minimale d'un circuit de courant entier due à la perte thermique de courant liée à une impulsion de courant, un maximum le plus petit possible de l'amplitude de l'intensité du courant de la bobine, un maximum le plus petit possible de l'amplitude de la tension de la bobine, un maximum le plus petit possible de l'amplitude de la pente temporelle du courant de la bobine, un maximum le plus petit possible de l'amplitude de la pente temporelle de la tension de la bobine, une énergie minimale de perte de courant de Foucault dans le tissu corporel liée à une impulsion de courant, un effet de stimulation maximale à une valeur maximale prédéterminée de la tension de la bobine, un effet de stimulation maximale à une énergie prédéterminée stockée dans un ou plusieurs condensateurs de stockage ou le niveau de bruit de la bobine causé par l'artéfact acoustique.

**Figur 1**

**Figur 2**

**Figur 3**

**Figur 4**

Amplitude                    Vollwellen-Puls

1,0

0,75                                         Spulenstrom

0,5

0,25

0        T/4        T/2        3T/4        T        Zeit

-0,25

-0,5

Spulenspannung

-0,75

-1,0

**Figur 5**

210

620

220

Thyristor I          Thyristor II

Ladeschaltung    Kondensator              610          260

Spule

**Figur 6**

**Figur 7**

**Figur 8**

**Figur 9**

Verhältnis der Dauer der 1.Halbwelle zur Dauer der 2.Halbwelle

**Figur 10**

1110 Noise
1190
1140 Nonlinear Filter
1150 LP
1160 Analysis

1120 Stimulator (Oscillator with m DOF)
1130 Lin. Filter

n≤m Freiheitsgrade

1170 Control System

Signal Locked

1180 Assessment

→ Pulsenergie
→ Spulenverluste
→ maximaler Spulenstrom
→ maximale Spulenspannung
→ maximale Spannungssteilheit
→ ...

**Figur 11**

1210

1220

1230

**Figur 12**

**Figur 13**

**Figur 14**

**Figur 15**

**Figur 16**

1710

1720

**Figur 17**

1810                    1820

| Stromrichter | Glättungs-Filter |
| | |

260

Stimulationsspule

**Figur 18**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007145838 A2
- US 2009018384 A1
- EP 1894600 A1
- DE 3524232 A1
- DE 10103031, von R. Marquardt

**In der Beschreibung aufgeführte Nicht-Patentliteratur**

- **R. SIEBNER ; U. ZIEMANN.** Das TMS-Buch. Springer Verlag
- **A. V. PETERCHEV ; R. JALINOUS ; S. H. LISANBY.** A Transcranial Magnetic Stimulator Inducing Near-Rectangular Pulses With Controllable Pulse Width (cTMS). *IEEE Transactions on Biomedical Engineering,* 2008, vol. 55 (1
- Analysis of a novel magnetic stimulation system: Magnetic harmonic multicycle stimulation (MHMS). **GOETZ S M et al.** BIOMEDICAL AND PHARMACEUTICAL ENGINEERING, 2009. ICBPE '09. INTERNATIONAL CONFERENCE ON. IEEE, Dezember 2009, vol. 2, 1-6
- **A. L. HODGKIN ; A. F. HUXLEY.** A Quantitative Description of Membrane Current and its Application to Conduction and Excitation in Nerve. *Journal of Physiology,* 1952, vol. 117, 500-544